(19) Europäisches Patentamt
European Patent Office
Office européen des brevets

(11) Numéro de publication: **0 313 960 B1**

(12) # FASCICULE DE BREVET EUROPEEN

(45) Date de publication de fascicule du brevet: **29.07.92**

(51) Int. Cl.$^5$: **C07J 1/00**, C07J 41/00, C07J 51/00

(21) Numéro de dépôt: **88117295.1**

(22) Date de dépôt: **22.08.85**

(60) Numéro de publication de la demande initiale en application de l'article 76 CBE : **0 176 399**

(54) **Nouveaux stéroides substitués en position 10 par un radical comportant une double ou triple liaison et leur procédé de préparation.**

(30) Priorité: **24.08.84 FR 8413189**

(43) Date de publication de la demande:
**03.05.89 Bulletin 89/18**

(45) Mention de la délivrance du brevet:
**29.07.92 Bulletin 92/31**

(84) Etats contractants désignés:
**AT BE CH DE FR GB IT LI LU NL SE**

(56) Documents cités:
**EP-A- 0 023 856**
**GB-A- 2 078 749**

(73) Titulaire: **ROUSSEL-UCLAF**
**35, Boulevard des Invalides**
**F-75007 Paris(FR)**

(72) Inventeur: **Torelli, Vesperto**
**5, rue de la Convention**
**F-94700 Maisons-Alfort(FR)**
Inventeur: **Nedelec, Lucien**
**45 Boulevard de l'Ouest**
**F-93340 Le Raincy(FR)**
Inventeur: **Moguilewsky, Martine**
**37, rue Lamartine**
**F-75009 Paris(FR)**
Inventeur: **Moura, Anne-Marie**
**15-29, rue Guilleminot**
**F-75014 Paris(FR)**

(74) Mandataire: **Fritel, Hubert et al**
**111, route de Noisy B.P. no 9**
**F-93230 Romainville(FR)**

EP 0 313 960 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

Rank Xerox (UK) Business Services

**Description**

La présente invention concerne de nouveaux stéroïdes substitués en position 10 par un radical comportant une double ou triple liaison et leur procédé de préparation.

Dans le brevet européen n° 0.023.856 est décrit un produit, le 3,3-éthylènedioxy 10β-éthynyl 5α-hydroxy estra 9(11)ène 17-one, du même type que les produits objet de la présente demande. Cependant ce produit est utile à la synthèse de produits finals différents de ceux que les intermédiaires nouveaux de la présente demande servent à préparer.

L'invention a pour objet les produits de formule générale II :

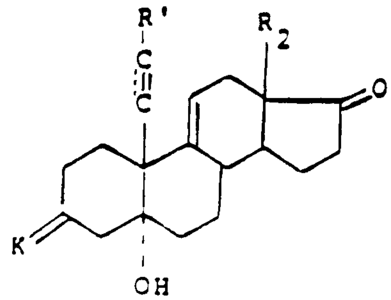

(II)

dans laquelle :

- ou bien R' représente R, R représentant :
- soit un atome d'hydrogène,
- soit un radical alkyle, alkényle ou alkynyle ayant au plus 8 atomes de carbone, éventuellement substitué par les radicaux hydroxyle, carboxy éventuellement estérifié, amino, tritylamino, chloroacétylamino, trifluoroacétylamino ; trichloroéthoxycarbonylamino, monoalkyl ou dialkylamino ou halogènes,
- soit un radical aryle ou aralkyle éventuellement substitué par les radicaux hydroxyle, carboxy éventuellement estérifié, amino, mono ou dialkylamino, alkyle, alkoxy ou alkylthio,
- soit un radical hydroxyle, tétrahydropyrannyloxy, tert-butyloxy, carboxy éventuellement estérifié, amino, mono ou dialkylamino, tritylamino, chloroacétylamino, trifluoroacétylamino ou trichloroéthoxycarbonylamino,
- soit un atome d'halogène,
- soit un radical trialkylsilyle,

ou bien R' représente le substituant R dans lequel les fonctions réactives sont protégées.

$R_2$ représente un radical méthyle ou éthyle.

K représente un groupement protecteur de la fonction cétone, étant entendu que R' ne peut pas représenter un atome d'hydrogène lorque $R_2$ représente un radical méthyle, K représente un radical éthylènedioxy et le trait pointillé sur le substituant R-C≡C- indique la présence d'une troisième liaison entre les carbone qui les portent.

L'invention a plus particulièrement pour objet les produits de formule générale II telle que définie ci-dessus dans laquelle R' est choisi dans le groupe formé par un atone d'hydrogène, un radical alkyle ayant de 1 à 4 atomes de carbone, un radical hydroxyméthyle, un radical tétrahydropyrannyloxyméthyle et un radical phényle et K est choisi dans le groupe formé par les cétals tels que le bis méthoxy, les cétals cycliques tels que l'éthylène dioxy, les oximes et les alkyloximes.

Les produits de formule II sont des intermédiaires pour la préparation des produits de formule générale I :

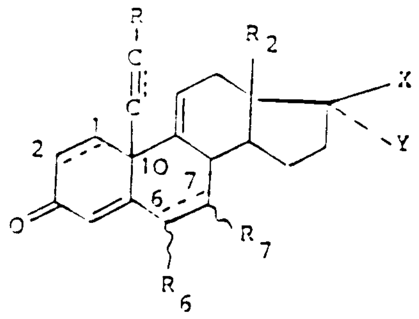

(I)

dans laquelle R représente :

- soit un atome d'hydrogène,
- soit un radical alkyle, alkényle ou alkynyle ayant au plus 8 atomes de carbone, éventuellement substitué par les radicaux hydroxyle, carboxy éventuellement estérifié, amino, tritylamino, chloroacétylamino, trifluoroacétylamino; trichloroéthoxycarbonylamino, monoalkyl ou dialkylamino ou halogènes,
- soit un radical aryle ou aralkyle éventuellement substitué par les radicaux hydroxyle, carboxy éventuellement estérifié, amino, mono ou dialkylamino, alkyle, alkoxy ou alkylthio,
- soit un radical hydroxyle, tétrahydropyrannyloxy, tert-butyloxy, carboxy éventuellement estérifié, amino, mono ou dialkylamino, tritylamino, chloroacétylamino, trifluoroacétylamino ou trichloroéthoxycarbonylamino,
- soit un atome d'halogène,
- soit un radical trialkylsilyle,

$R_6$ et $R_7$ sont tels que soit $R_6$ et $R_7$ forment avec les carbones qui les portent un radical cyclopropyle, soit $R_6$ représente un atome d'hydrogène et $R_7$ représente le radical $R_1$, $R_1$ représentant :

- soit un atome d'hydrogène,
- soit un radical alkyle, alkényle ou alkynyle ayant au plus 6 atomes de carbone éventuellement substitué par les radicaux hydroxyle, carboxy éventuellement estérifié, amino, tritylamino, chloroacétylamino, trifluoroacétylamino, trichloroéthoxycarbonylamino, monoalkyl ou dialkylamino ou halogènes.

$R_2$ représente un radical méthyle ou éthyle,

X et Y sont tels que :

- soit X représente un radical hydroxyle, acétyloxy, propionyloxy, méthoxy ou éthoxy et Y représente ou bien un radical $-CH_2CH_2CO_2M$ dans lequel M représente un atome d'hydrogène un atome de métal alcalin ou un radical ammonium,
  ou bien un radical $-CH_2CH_2-CH_2OH$,
- soit X et Y représentent ensemble un radical :

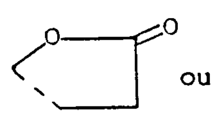 ou 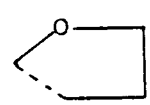

- soit X et Y représentent ensemble un radical

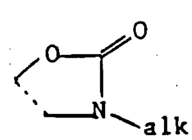

dans lequel alk représente un radical alkyle renfermant au plus 8 atomes de carbone,

- soit X et Y représentent ensemble un radical

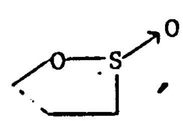,

ou X représente un radical hydroxyle et Y un radical

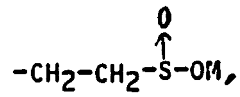

M étant défini comme ci-dessus,

- soit X représente un radical hydroxyle, acétyloxy, propionyloxy, méthoxy ou éthoxy et Y représente, ou bien un atome d'hydrogène ou bien Y représente $R_4$, $R_4$ représente un radical alkyle, alkényle ou alkynyle ayant au plus 6 atomes de carbone,

  les traits pointillés en position 1 (2) et 6 (7) indiquent la présence éventuelle d'une seconde liaison entre les carbones qui les portent, étant entendu qu'il ne peut pas y avoir de seconde liaison en position 6 (7) lorsque $R_6$ et $R_7$ forment avec les carbones qui les portent un radical cyclopropyle, le trait pointillé sur le substituant R-C≡C- en position 10 indique la présence éventuelle d'une troisième liaison entre les carbones qui les portent,

  les traits ondulés en position 6 et 7 indiquent que les substituants $R_6$ et $R_7$ peuvent se trouver dans l'une des positions possibles ou ,

  étant entendu que R ne peut pas représenter un atome d'hydrogène lorsque $R_6$ et $R_7$ représentent chacun un atome d'hydrogène,

  $R_2$ représente un radical méthyle, X représente un radical hydroxy ou acétoxy et Y un atome d'hydrogène, les traits pointillés en position 1 (2) et 6 (7) ne représentent pas une seconde liaison entre les carbones qui les portent et le trait pointillé sur le substituant en position 10 indique la présence d'une troisième liaison entre les carbones qui le portent.

Les produits de formule I sont décrits et revendiqués dans le brevet européen EP 0.176.399 dont la présente demande constitue une division.

Parmi les valeurs de R on peut citer les radicaux méthyle, éthyle, propyle, isopropyle, butyle, butyle secondaire, tert-butyle et les radicaux pentyle, hexyle, heptyle ou octyle éventuellement ramifiés.

On peut également citer les radicaux alkényles suivants : vinyle, allyle, 1-propényle, butényle, les radicaux alkynyles tels que éthynyle, propargyle ou butynyle.

Ces différents radicaux peuvent être substitués, par exemple par les radicaux hydroxyle, carboxy éventuellement estérifié tel que méthoxycarbonyle, éthoxycarbonyle, amino éventuellement protégé tel que tritylamino, chloroacétylamino, trifluoroacétylamino, trichloroéthoxycarbonylamino, monoalkyl ou dialkylamino tel que méthylamino et diméthylamino, les atomes d'halogènes,de préférence chlore ou brome.

Les radicaux aryles et aralkyles que peut également représenter R sont de préférence un radical phényle, benzyle ou phényléthyle. Ces radicaux peuvent également être substitués, par exemple par les radicaux hydroxyle, carboxy éventuellement estérifiés, amino, mono ou dialkylamino, alkyle tel que méthyle, alkoxy tel que méthoxy, alkylthio tel que méthylthio.

La valeur hydroxyle protégée que peut représenter R peut être par exemple un radical tétrahydropyranyloxy ou tert-butyloxy.

La valeur trialkylsilyle préférée est la valeur triméthylsilyle.

L'invention a également pour objet un procédé de préparation des produits de formule générale II telle que définie ci-dessus, caractérisé en ce que

1- soit l'on fait agir un lithien de formule R'-C≡C-Li sur un produit de formule (III) :

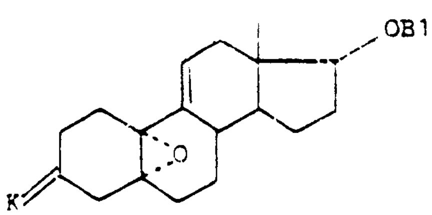

(III)

dans lequel $B_1$ représente un groupement protecteur du radical hydroxyle pour obtenir un produit de formule (IV) :

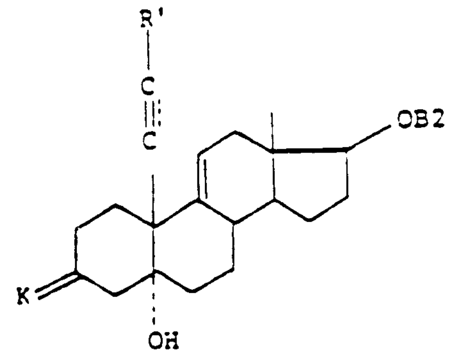

(IV)

dans laquelle $B_2$ représente un atome d'hydrogène ou le radical $B_1$ précédent, produit que l'on soumet d'abord à une réaction de déprotection du radical hydroxyle si le groupement protecteur n'a pas été clivé lors de la réaction précédente puis à une réaction d'oxydation pour obtenir les produits de formule II ;

2- soit en faisant agir un magnésien de formule R'-C≡C-MgX sur un produit de formule ($III_1$) :

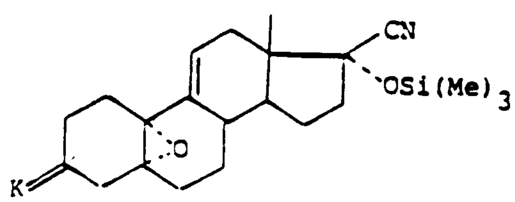

($III_1$)

pour obtenir un produit de formule ($IV_1$) :

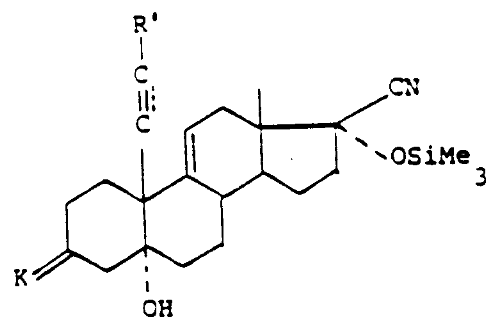

($IV_1$)

produit qui par hydrolyse alcaline donne le produit de formule (II) attendu.

Dans le procédé ci-dessus,

1) Le groupement protecteur que peut représenter $B_1$ est de préférence un groupement acyle tel que benzoyle. Le lithien R'-C≡C-Li peut être préparé par les méthodes usuelles ; l'hydrolyse du groupement $B_1$ est également réalisée par les méthodes usuelles si le clivage n'est pas déjà intervenu lors de la réaction précédente. On peut utiliser une hydrolyse en milieu basique. L'oxydation en position 17 est effectuée par exemple par utilisation de chlorochromate de pyridinium préparé selon Tet. Letters. n°31 (1975) p. 2647. Un tel exemple de préparation est décrit dans le brevet européen 0.023.856 (ex. 5).

2) L'action du magnésien de formule R'-C≡C-MgX sur le produit de formule $III_1$ est effectuée dans les conditions usuelles. Lorsque le trait pointillé représente une troisième liaison entre les carbones, le magnésien peut être préparé in situ par action du produit R'-C≡C-H sur un magnésien tel que le bromure d'éthylmagnésium. On opère alors dans un solvant tel que le tétrahydrofuranne ou l'éther éthylique. L'hydrolyse du produit de formule $IV_1$ obtenu est effectuée dans les conditions usuelles. Des exemples de telles préparations figurent ci-après dans la partie expérimentale.

Les produits de formules III et $III_1$ sont soit connus, par exemple par le brevet français 2.377417, soit peuvent être préparés par des méthodes usuelles à partir de ces produits connus.

Les produits de formule II de la présente demande trouvent leur application dans la synthèse des produits de formule I telle qu'indiquée ci-dessus. Ce procédé est caractérisé en ce que l'on traite un produit

de formule II :

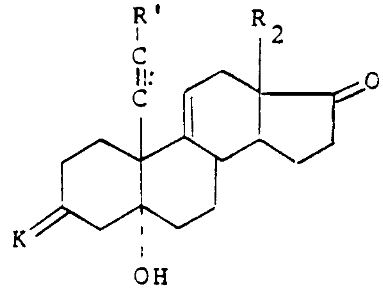

II

dans laquelle R' représente soit R, R ayant la signification indiquée ci-dessus soit le substituant R dans lequel les fonctions réactivées sont protégées, $R_2$ a la signification indiquée ci-dessus et K représente un groupement protecteur de la fonction cétone,

I soit d'abord par un halogénure de triméthylsulfonium en présence d'une base forte puis par un dérivé métallique de l'acétonitrile et enfin par une base puis par un acide,

soit d'abord par un réactif de formule :

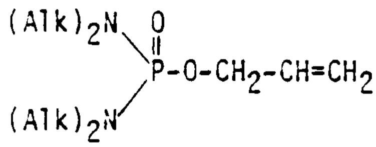

dans lequel Alk représente un radical alkyle ayant de 1 à 4 atomes de carbone en présence d'une base forte puis par un acide pour obtenir un produit de formule $I_A$ :

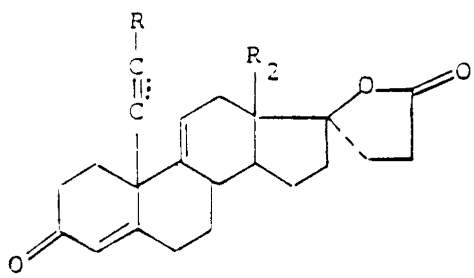

$I_A$

II- soit par un réactif de formule :

$XMg\text{-}CH_2CH_2CH_2OB$

dans lequel X représente un atome d'halogène et B représente un groupement protecteur du radical hydroxyle ou un alcoolate de magnésium pour obtenir après traitement par un acide un produit de formule $I'_A$ :

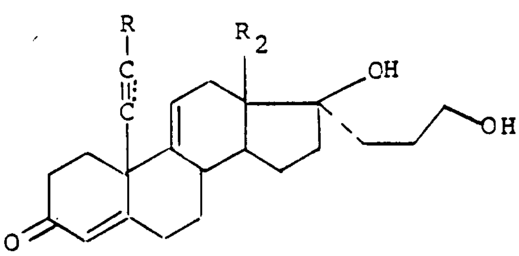

$I'_A$

produit de formule $I'_A$ que l'on traite éventuellement par un halogénure d'acide sulfonique en présence

d'une base pour obtenir un produit de formule $I''_A$ :

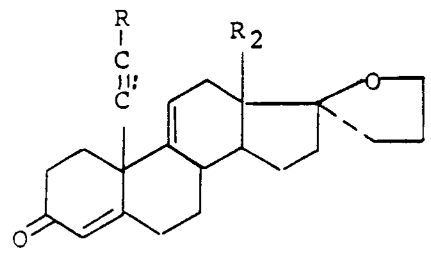

$I''_A$

III soit par un réactif de réduction puis par un acide pour obtenir un produit de formule $I_{3A}$ :

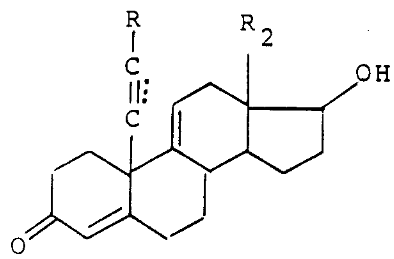

$I_{3A}$

IV soit par un organo métallique $R_4$ MgX ou $(R_4)_2$-CuLi, X représentant un atome d'halogène, puis par un acide, pour obtenir un produit de formule $I_{4A}$ :

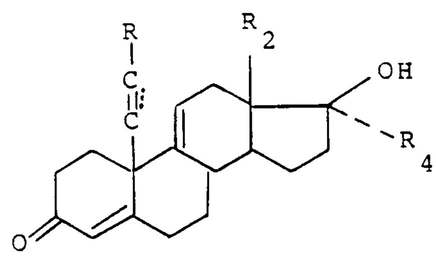

$I_{4A}$

V soit d'abord par un halogénure de triméthylsulfonium en présence d'une base forte, puis par une amine primaire de formule $H_2N$-alk, alk étant défini comme précédemment, en présence d'un acide, par un chloroformiate d'alkyle, par une base forte et enfin par un acide, pour obtenir un produit de formule $I_{5A}$ :

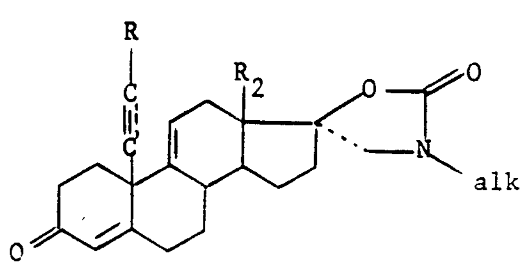

$I_{5A}$

VI soit d'abord par un halogénure de triméthylsulfonium en présence d'une base forte, puis par le méthyl terbutyl sulfoxyde en présence de n-butyllithium pour obtenir un produit de formule

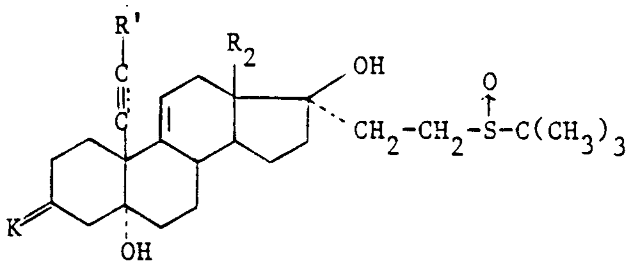

sous forme d'un mélange de deux diastéréoisomères au niveau de l'atome de soufre, sépare si désiré les deux diastéréoisomères, puis soumet soit leur mélange, soit chacun d'eux séparément, à l'action d'un acide, pour obtenir un produit de formule

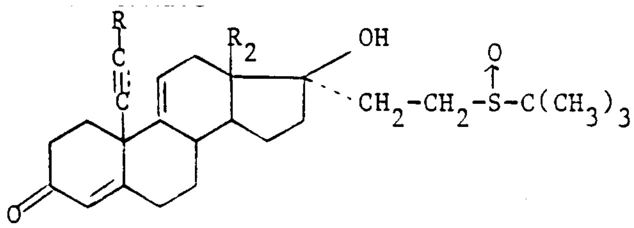

sous forme d'un mélange de deux diastéréoisomères ou un diastéréoisomère, sépare le cas échéant, les diastéréoisomères, puis soumet soit leur mélange, soit chacun d'eux séparément, à l'action du N-chloro ou du N-bromo succinimide, pour obtenir un produit de formule $I_{6A}$ :

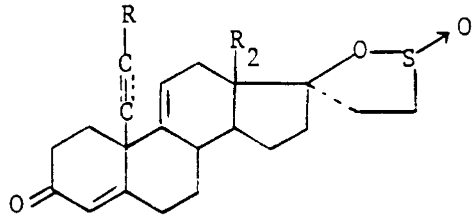

$I_{6A}$

et que, si désiré, l'on traite les produits de formules $I_A$, $I_A$, $I''_A$, $I_{3A}$, $I_{4A}$, $I_{5A}$ et $I_{6A}$ par un orthoformiate d'alkyle en présence d'un acide puis par un agent de déshydrogénation pour obtenir les produits de formule $I_B$, $I'_B$, $I''_B$, $I_{3B}$, $I_{4B}$, $I_{5B}$ et $I_{6B}$ de formules suivantes :

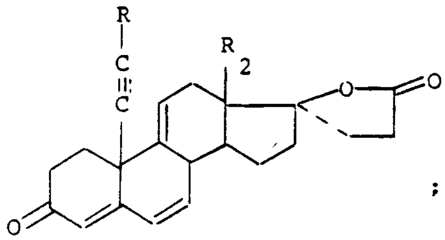

;

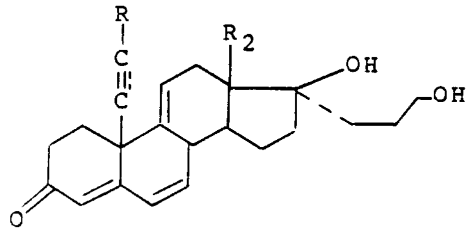

$I_B$ $I'_B$

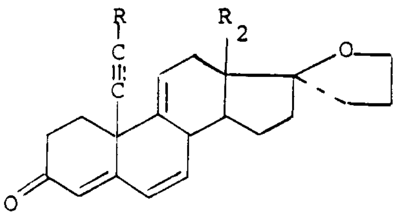

$I''_B$

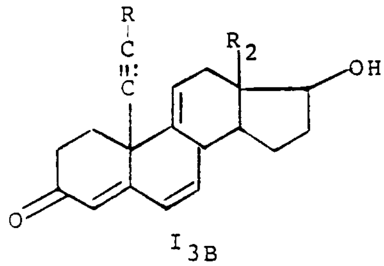

$I_{3B}$

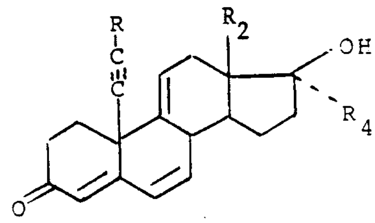

$I_{4B}$

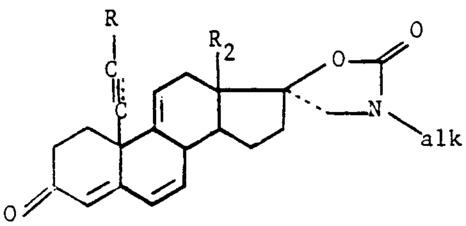

$I_{5B}$

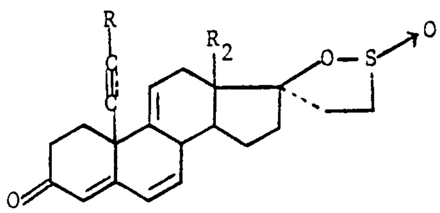

$I_{6B}$

produits de formules $I_B$, $I'_B$, $I''_B$, $I_{3B}$, $I_{4B}$, $I_{5B}$ et $I_{6B}$ que l'on traite si désiré :

- ou bien par un organo magnésien de formule $R_1$Mgx' éventuellement en présence d'un sel de cuivre, formule dans laquelle $R_1$ a la signification indiquée ci-dessus et X' représente un atome d'halogène
- ou bien par un organo métallique de formule $(R_1)_2$ CuLi puis par un acide, pour obtenir les produits de formules $I_C$, $I'_C$, $I''_C$, $I_{3C}$, $I_{4C}$, $I_{5C}$ et $I_{6C}$ :

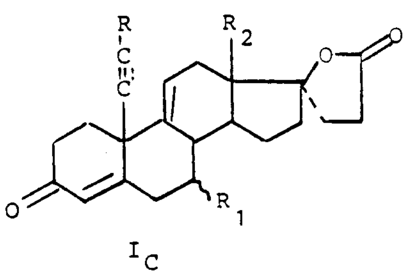

;

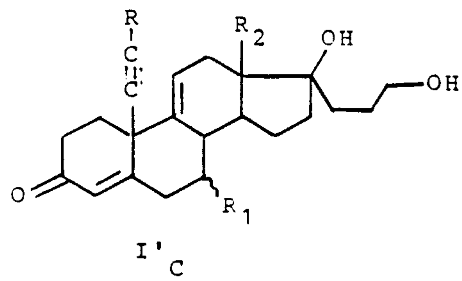

$I_C$ $I'_C$

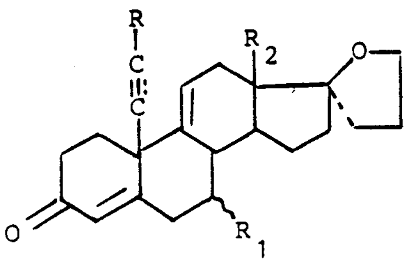

;

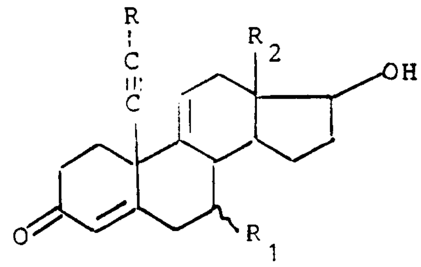

I"C

I3C

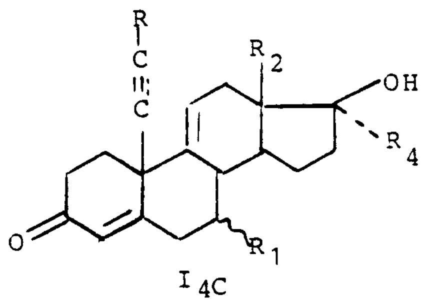

I4C

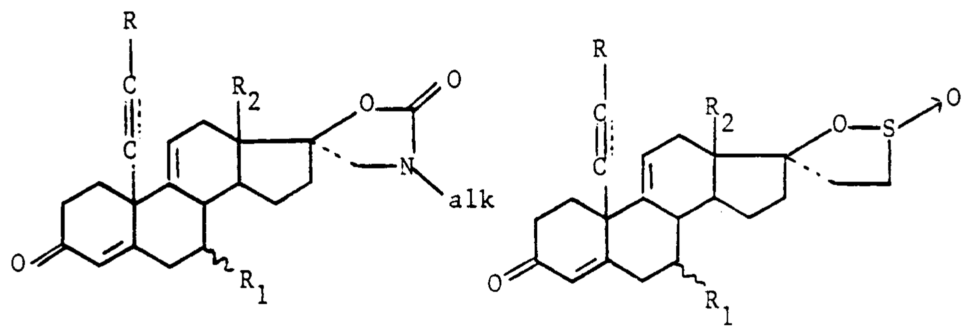

I5C

I6C

sous forme d'un mélange d'isomères 7α et 7β, mélange que,si désiré, l'on sépare et que,si désiré,l'on soumet les produits 7β à un réactif de déshydrogénation pour obtenir les produits Δ6 (7) correspondants;

- ou bien l'on soumet les produits de formules $I_B$, $I'_B$, $I''_B$, $I_{3B}$, $I_{4B}$, $I_{5B}$ et $I_{6B}$ à l'action d'un réactif choisi dans le groupe constitué par l' iodure de triméthylsulfonium et l'iodure de triméthylsulfoxonium en présence d'une base forte pour obtenir les produits de formules $I_D$, $I'_D$, $I''_D$, $I_{3D}$, $I_{4D}$, $I_{5D}$ et $I_{6D}$ :

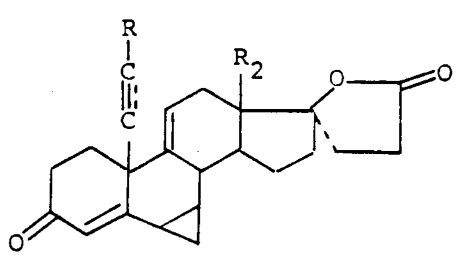

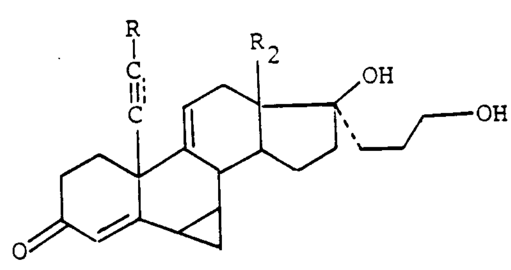

ID

I'D

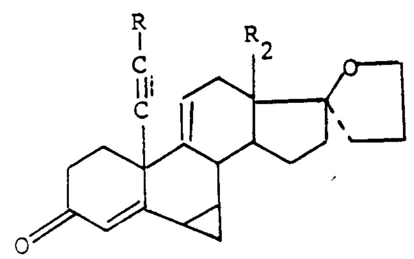

I"$_D$

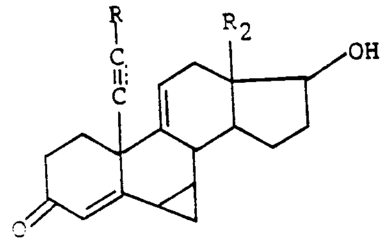

I$_{3D}$

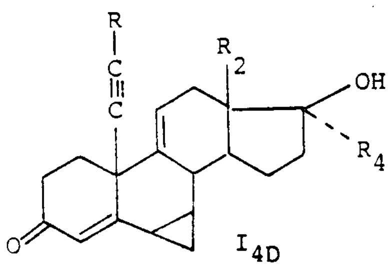

I$_{4D}$

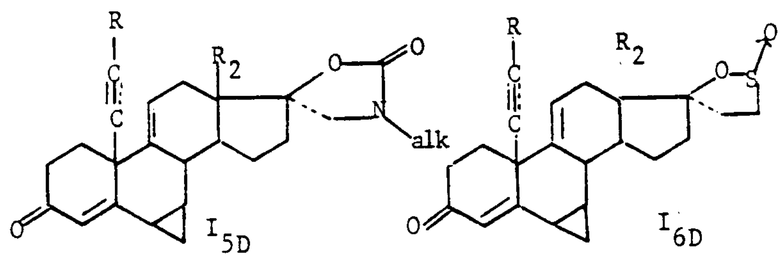

I$_{5D}$ I$_{6D}$

sous forme d'un mélange 6α, 7α et 6β, 7β et sépare, si désiré, les isomères ainsi obtenus et que si désiré l'on traite les produits $I_A$ $I'_A$, $I''_A$, $I_{3A}$, $I_{4A}$, $I_{5A}$, $I_{6A}$, $I_B$,$I'_B$, $I''_B$, $I_{3B}$, $I_{4B}$, $I_{5B}$, $I_{6B}$, $I_C$, $I'_C$, $I''_C$, $I_{3C}$, $I_{4C}$, $I_{5C}$, I6C, $_D$, $I'_D$, $I''_D$, $I_{3D}$, $1_{4D}$, $I_{5D}$, $I_{6D}$, par un réactif de déshydrogénation ou par un microorganisme susceptible de déshydrogéner la molécule en position 1 (2) pour obtenir les produits correspondants comportant une insaturation en position 1 (2) et que,si désiré,l'on traite les produits de formules $I_A$, $I_B$, $I_C$ et $I_D$ et les produits correspondants comportant une insaturation en position 1 (2) à l'aide d'un hydroxyde alcalin ou de l'ammoniaque pour obtenir les produits correspondants dans lesquels X représente un radical hydroxyle et Y représente un radical $-CH_2CH_2CO_2M'$ dans lequel M' représente un atome de métalalcalin ou un radical ammonium et,si désiré,soumet les produits ainsiobtenus à l'actiond'un agent acide pour obtenir les produits dans lesquels X représente un radical hydroxyle et Y représente un radical-$CH_2CH_2CO_2H$, et que si désiré l'on traite les produits dans lesquels le substituant en position 10 comporte un substituant R du type hydroxyalkyle, par un tétra-bromure ou tétrachlorure de carbone en présence de triphénylphosphine, pour obtenir les dérivés bromés et chlorés correspondants, et que si désiré, l'on traite les produits dans lesquels le substituant en position 10 comporte une triple liaison par un agent d'hydrogénation sélective pour obtenir les produits correspondants dans lesquels le substituant en position 10 comporte une double liaison et que si désiré, l'on traite les produits comportant en position 10 un substituant éthynyl par un halo-succinimide pour obtenir les produits correspondant comportant en position 10 un substituant -C≡C-Hal, et que si désiré l'on acyle ou éthérifie le radical hydroxyle en position 17β des produits comportant en position 17α un atome d'hydrogène, un radical $R_4$ ou un radical $-CH_2-CH_2-CH_2OH$.

Les groupements protecteurs des fonctions que peut comporter R, par exemple les groupements hydroxy ou amino, sont choisis parmi les groupements connus.

Le groupement protecteur de la fonction cétone que représente K peut être un cétal, un thiocétal, un oxime ou un alkoxime. On utilise de préférence un cétal tel que le bis méthoxy ou un cétal cyclique tel que l'éthylène dioxy. Ces groupements sont éliminés en milieu acide.

L'halogénure de triméthylsulfonium que l'on utilise de préférence est l'iodure. La base forte en présence de laquelle on agit est de préférence le terbutylate de potassium.

On fait ensuite agir un dérivé métallique de l'acétonitrile qui peut par exemple être le dérivé lithien préparé en présence de butyllithium. Le dérivé ainsi obtenu qui comporte en position 17 un radical cyanoéthyle est ensuite soumis à l'action d'une base qui peut être par exemple la soude ou la potasse et on acidifie le produit obtenu par l'acide chlorhydrique de préférence.

L'action du tétralkyl phosphorodiamidate d'allyle, de préférence le tétraméthyle, sur le produit de formule II est effectuée selon la méthode décrite par STURTZ et YAOUANC, Synthesis 1980 p. 289. La base forte en présence de laquelle on agit est de préférence le butyllithium On peut également opérer en plus en présence de diazabicyclo octane (DABCO) ou d'un éther couronne. On acidifie ensuite à l'aide d'acide chlorhydrique, de préférence.

L'action du produit de formule $XMg-CH_2CH_2CH_2OB$ sur le produit de formule II est effectuée selon les

méthodes classiques de préparation de magnésien. Le radical B peut représenter un groupement protecteur habituel du radical hydroxyle, tel que le tétrahydropyrannyle ou le ter-butyle. B peut également représenter un sel de magnésium tel que MgCl. La préparation et l'utilisation d'un tel groupement sont décrites par CAHIEZ, ALEXAKIS et NORMANT Tetr. lett. n° 33, 1978 p. 3013. On acidifie ensuite à l'aide d'un acide, l'acide chlorhydrique de préférence.

La transformation des produits de formule $I'_A$ en produits $I''_A$ est effectuée de préférence en présence de chlorure de tosyle et d'une base aminée telle que la pyridine.

La réduction des produits de formule II en produits de formule $I_{3A}$ est effectuée par les méthodes classiques. On peut utiliser par exemple un hydrure tel que le borohydrure de sodium.

L'hydrolyse du groupement protecteur K est effectuée de manière générale par les méthodes classiques en milieu acide par exemple, par addition d'acide chlorhydrique.

L'addition du magnésien $R_4MgX$ et la réaction avec $(R_4)_2CuLi$ sont effectuées selon les méthodes classiques,on opère par exemple dans un solvant anhydre tel que le tétrahydrofuranne ou l'éther éthylique et à une température inférieure à la température ambiante. Comme pour les autres réactions indiquées précédemment on fait agir ensuite un acide tel que l'acide chlorhydrique pour régénérer la fonction cétone en position 3.

La base forte utilisée dans la cyclisation conduisant au produit $I_{5A}$ est de préférence la potasse méthanolique.

L'acide en présence duquel est effectuée la réaction avec l'amine est de préférence l'acide paratoluènesulfonique.

L'halogénure de triméthylsulfonium est de préférence un iodure et la base forte utilisée est la soude, la potasse ou le terbutylate de potassium.

La séparation des diastéréoisomères est effectuée par les moyens classiques de chromatographie et de cristallisation.

L'action d'un orthoformiate sur les produits $I_A$, $I'_A$, $I''_A$, $I_{3A}$, $I_{4A}$, $I_{5A}$, et $I_{6A}$, a pour but de préparer les produits 3-alkoxy Δ3,5 de formules :

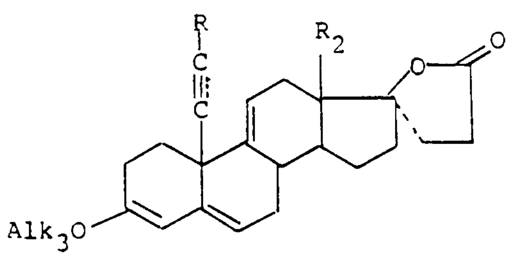

$I_{OA}$

;

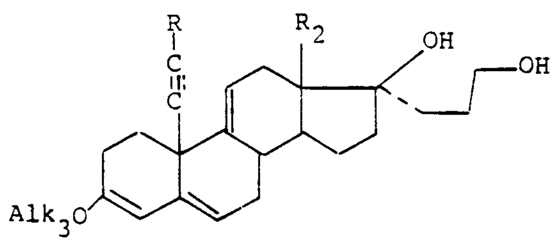

$I'_{OA}$

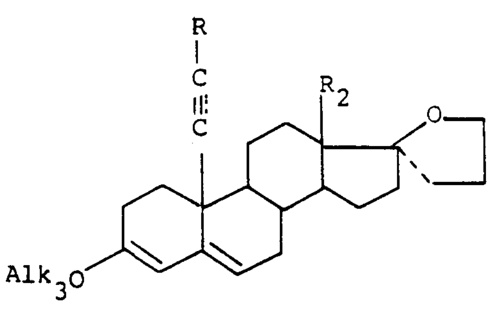

$I''_{OA}$

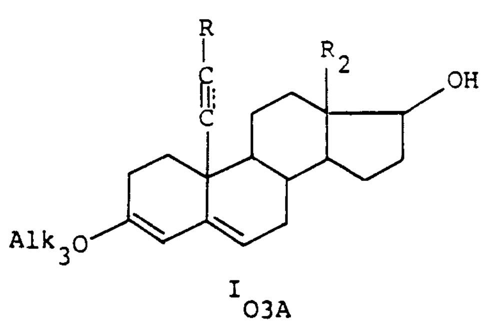

$I_{O3A}$

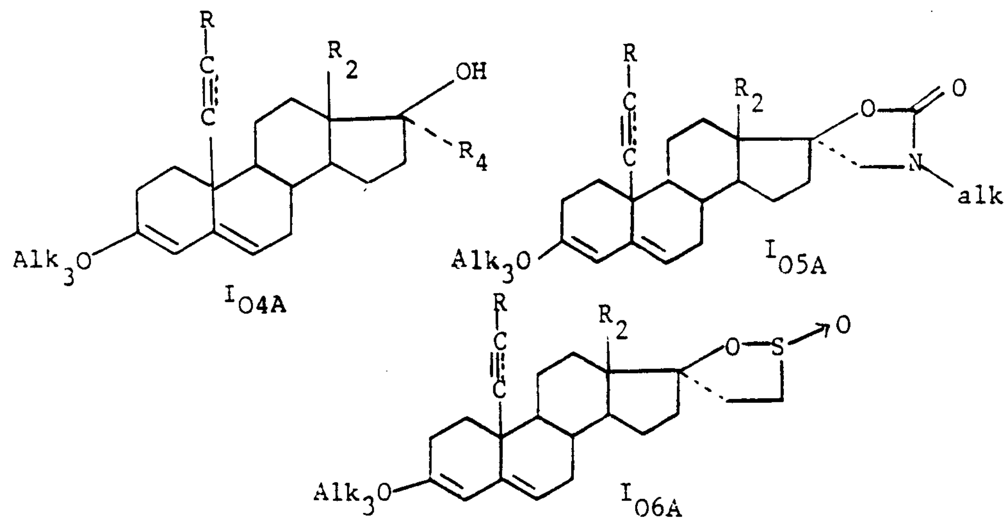

$I_{04A}$ $I_{05A}$ $I_{06A}$

Dans les formules $I_{0A}$, $I'_{0A}$, $I''_{0A}$, $I_{03A}$, $I_{04A}$, $I_{05A}$ et $I_{06A}$ les radicaux R, $R_2$ et $R_4$ ont la signification précédente, $Alk_3$ représente un radical alkyle ayant de 1 à 4 atomes de carbone. L'orthoformiate utilisé est de préférence l'orthoformiate d'éthyle en présence d'acide paratoluènesulfonique. L'agent de déshydrogénation que l'on utilise est de préférence le chloranile (tétrachloro 1,4-benzoquinone). On peut également utiliser le DDQ (2,3-dichloro 5,6-dicyano 1,4-benzoquinone). L'action du produit de formule $R_1MgX'$ dans laquelle X' représente un atome de chlore, brome ou iode est effectuée de préférence en présence d'un sel cuivrique tel que l'acétate, le chlorure, le bromure cuivrique ou un sel cuivreux tel que le chlorure, le bromure ou l'iodure cuivreux.

L'acide que l'on utilise après action d'un produit de formule $(R_1)_2CuLi$ est l'acide chlorhydrique, nitrique ou sulfurique.

La séparation éventuelle des différents isomères est réalisée par chromatographie ou par cristallisation fractionnée. La déshydrogénation éventuelle des produits 7$\beta$ est effectuée dans les conditions énoncées précédemment.

La base utilisée pour former l'ylide correspondant à l'iodure de triméthylsulfonium et l'iodure de triméthylsulfoxonium est l'hydrure de sodium ou le tert-butylate de potassium.

La séparation éventuelle est effectuée selon les méthodes classiques énoncées ci-dessus.

La transformation des produits $I_A$ $I_{6A}$, $I_B$ à $I_{6B}$, $I_C$ à $I_{6C}$ et $I_D$ à $I_{6D}$ en produits comportant une insaturation en position 1 (2) est effectuée de préférence en faisant agir par voie biochimique, la bactérie "Arthrobacter simplex". On peut également utiliser un autre microorganisme. Dans ce cas, la réaction est effectuée de préférence en milieu aqueux tamponné.

On peut également utiliser la voie chimique en soumettant les produits à l'action d'un dérivé de la p-benzoquinone ou du chloranile, la réaction a lieu au sein, par exemple, d'une solution dans un solvant organique tel que le dioxanne, l'acétone, le benzène ou l'alcool tertbutylique.

On peut également utiliser, comme réactif de déshydrogénation, l'anhydride sélénieux ou benzène sélénique.

L'hydroxyde alcalin auquel on soumet éventuellement les produits de formules $I_A$, $I_B$ et $I_C$ ainsi que les produits correspondants comportant une insaturation en position 1 (2) est de préférence la soude ou la potasse.

L'agent acide auquel on soumet éventuellement les produits ainsi obtenus est l'acide chlorhydrique, sulfurique, nitrique ou acétique.

L'agent d'hydrogénation sélective auquel on soumet éventuellement les produits comportant en position 10 un substituant ayant une triple liaison est de préférence l'hydrogène en présence d'un catalyseur tel que le catalyseur de Lindlar.

L'halosuccinimide que l'on fait agir avec les produits comportant en position 10 un substituant éthynyl est le N-bromo ou le N-chlorosuccinimide. On opère de préférence en présence de nitrate d'argent.

L'acylation éventuelle des produits comportant en position 17 un radical hydroxyle et en position 17$\beta$ un atome d'hydrogène ou un radical $R_4$ est effectuée selon les méthodes usuelles. On peut utiliser par exemple un anhydride ou un halogénure d'acyle tel que l'anhydride acétique ou le chlorure d'acétyle. L'éthérification des mêmes produits est effectuée également dans les conditions usuelles par exemple à l'aide d'un halogénure d'alkyle.

Les produits de formule I telle que définie ci-dessus ainsi que la 10β-éthynyl 17β-hydroxy estra 4,9 (11)-dien-3-one et la 10β-éthynyl 17β-acétoxy estra 4,9 (11)-dièn-3-one, cet ensemble constituant la formule générale I', présentent de très intéressantes propriétés pharmacologiques ; ils sont en particulier des antagonistes de l'aldostérone et augmentent la diurèse hydrosodée avec conservation du potassium organique.

Les produits de formule (I') offrent de plus l'avantage de ne présenter que très peu d'effets hormonaux secondaires.

Des tests effectués sur récepteur et in vivo ont, en particulier, montré que les produits de formule (I') sont moins anti-androgènes que des produits anti-aldostérone décrits précédemment.

Les produits de formule (I') peuvent donc être utilisés avantageusement pour lutter, notamment, contre l'hypertension artérielle et les insuffisances cardiaques.

La posologie utile varie en fontion de l'affection à traiter et de la voie d'administration ; elle peut aller par exemple de 5 mg à 500 mg et de préférence de 10 à 200 mg par jour chez l'adulte par voie orale pour le produit de l'exemple 1.

Les composés de formule (I') sont utilisés par voie buccale, rectale, transcutanée, ou intraveineuse. Ils peuvent être prescrits sous forme de comprimés, de comprimés enrobés, de cachets, de capsules, de granulés, d'émulsions, de sirops, de suppositoires et de préparations injectables.

Les exemples suivants illustrent l' invention sans toutefois la limiter.

Exemple 1 : γ-lactone de l'acide 10β-éthynyl 17β-hydroxy 3-oxo 19-nor 17α-pregna 4,9 (11)-diène 21-carboxylique :

Stade A : (17S) 3,3-éthylène dioxy 10β-éthynyl spiro/estr 9 (11)-ène 17, 2'-oxiran/5α-ol :

On ajoute sous agitation une solution de 3,06 g d'iodure de triméthylsulfonium dans 60 cm3 de diméthylsulfoxyde à une suspension de 4,25 g de 3,3 éthylène dioxy 10β-éthynyl 5α-hydroxy estra 9 (11) èn 17-one dans 60 cm3 de tétrahydrofuranne et 12 cm3 d'une solution 2,4 M de tertiobutylate de potassium dans le tétrahydrofuranne. Après une heure de réaction on verse le mélange réactionnel dans de l'eau additionnée de chlorure d'ammonium. On extrait à l'éther éthylique, lave la phase organique, sèche et obtient 4,5 g de produit attendu après évaporation du solvant sous pression réduite. Le produit est empâté dans de l'hexane, on essore les cristaux, les lave à l'hexane et obtient 3,895 g de produit attendu F : 142° C. Après recristallisation dans l'éthanol F : 144° C.

| Analyse : $C_{23}H_{30}O_4$ = 370,49 | | |
|---|---|---|
| Calculé : | C % : 74,56 | H % : 8,16 |
| Trouvé : | 74,7 | 8,2 |

Stade B : 5 α, 17β-dihydroxy 3,3-éthylènedioxy estr 9 (II)-èn-17α-yl 3-propionitrile :

On refroidit à -60° C une solution de 11 cm3 de N-isopropyl cyclohexyl amine dans 60 cm3 de tétrahydrofuranne anhydre et ajoute goutte à goutte 40 cm3 de n-butyl lithium en solution dans l'hexane (titre : 15 %) puis une solution de 3,5 cm3 d'acétonitrile dans 10 cm3 de tétrahydrofuranne anhydre. On obtient une suspension que l'on agite pendant 15 minutes à -5 -10° C. On introduit ensuite une solution de 4 g de produit obtenu au stade A dans 20 cm3 de tétrahydrofuranne. On rince avec S cm3 de tétrahydrofuranne puis laisse réagir à température ambiante pendant 20 heures. On verse le mélange dans une solution de chlorure d'ammonium et extrait à l'éther. On lave la phase organique à l'eau, sèche et obtient 9 g d'une huile après évaporation du solvant.

On filtre l'huile sur silice, élue avec un mélange cyclohexaneacétate d'éthyle (7-3) et sépare dans l'ordre 340 mg de produit de départ puis 5 g d'un produit que l'on cristallise dans l'éther au reflux. On refroidit, essore, lave à l'éther et obtient 2,827 g de produit attendu F : 200° C. Par recristallisation dans le chlorure de méthylène on obtient un échantillon analytique F : 203° C.

| Analyse : $C_{25}H_{33}O_4N$ = 411,54 | | | |
|---|---|---|---|
| Calculé :<br>Trouvé | C % : 72,96<br>72,8 | H % : 8,08<br>8,2 | N % : 3,4<br>3,4 |

Stade C : γ-lactone de l'acide 10β-éthynyl 17β-hydroxy 3-oxo 19-nor 17α-pregna 4,9 (11)-diène 21-carboxylique :

On chauffe au reflux pendant 4 heures trente une suspension de 1,9 g de produit obtenu au stade B dans 30 cm3 de méthanol avec 2 cm3 de potasse 6N. On refroidit et acidifie avec 3 cm3 d'acide chlorhydrique concentré. On dilue avec 15 cm3 de méthanol et chauffe de nouveau au reflux pendant une heure trente. On refroidit, dilue à l'eau et extrait à l'acétate d'éthyle. On obtient après lavage, séchage et évaporation de la phase organique 1,57 g de produit brut.

On filtre ce produit sur silice avec un mélange cyclohexane-acétate d'éthyle (1-1) et obtient 1,225 g de produit purifié que l'on empâte dans de l'éther isopropylique et essore. On obtient 1,17 g de produit attendu F : 210 °C.

Exemple 2 : 10β-éthynyl 17β-hydroxy 3-oxo estra-4,9 (11)-diène-17α-propanoate de potassium :

On porte au reflux pendant 15 minutes une suspension de 387 mg de produit obtenu à l'exemple 1 dans 4,3 cm3 de potasse méthanolique 0,246 N et 4,3 cm3 d'eau. On distille à sec à 50 °C sous pression réduite la solution obtenue puis élimine les traces d'eau par deux distillations d'éthanol absolu. La mousse obtenue est dissoute dans 2 cm3 d'éthanol à 95 °C, ajoute de l'éther lentement en grattant (environ 6 cm3), glace, essore, lave abondamment à l'éther puis sèche. On obtient 380 mg de produit F # 200 °C.

| Analyse : $C_{23}H_{27}O_4K$ = 406,57 | | |
|---|---|---|
| Calculé<br>Trouvé | C % : 61,22<br>: 61,4 | H % : 7,13<br>7,0 |

Exemple 3 : γ-lactone de l'acide 10β-éthényl 17β-hydroxy 3-oxo 19-nor 17α-pregna 4,9 (11) diène 21-carboxylique :

On dissout 180 mg de γ-lactone de l'acide 10β-éthynyl 17β-hydroxy 3-oxo 19-nor 17α-pregna 4,9 (11)-diène-21-carboxylique (préparé à l'exemple 1) dans 4 cm3 d'acétate d'éthyle avec 1 cm3 de pyridine, ajoute 19 mg de catalyseur de Lindlar et agite en atmosphère d'hydrogénation jusqu'à absorption de la quantité théorique d'hydrogène soit pendant 25 minutes environ. On filtre le catalyseur, le rince à l'acétate d'éthyle, dilue le filtrat avec de l'eau légèrement acidulée avec de l'acide chlorhydrique, décante la phase organique, lave à l'eau sèche et concentre à sec sous pression réduite. On chromatographie le résidu sur silice imprégnée de nitrate d'argent, élue par un mélange éther-hexane (4-1) recueille 150 mg de produit que l'on recristallise dans l'éther isopropylique pour obtenir 121 mg de produit attendu F = 144 °C.
$/\alpha/_D$ : -57 ° (c = 1 %, $CHCl_3$)

Exemple 4 : γ-lactone de l'acide 17β-hydroxy 3-oxo 10β-(1-propynyl) 19-nor 17α-pregna 4,9 (11)-diène-21-carboxylique :

Stade A : 3,3-éthylène dioxy 5 α-hydroxy 10β (1-propynyl) estr 9 (11)èn-17-one :

Dans 25 cm3 d'une solution éthérée de bromure d'éthylmagnésium 1 M diluée avec 25 cm3 de tétrahydrofuranne anhydre on fait barboter entre 0 et 5 °C du méthylacétylène pendant 1 heure. On ajoute 2,15 g de 3,3-éthylènedioxy 5 α, 10α-époxy 17α-triméthylsilyloxy estr-9 (11) ène 17β-carbonitrile dans 10 cm3 de tétrahydrofuranne,chauffe à 40 °C pendant 2 heures 30 et laisse 16 heures à température ambiante. On ajoute entre 0 et 5 °C, 20 cm3 d'une solution de chlorure d'ammonium, extrait au chlorure de méthylène, lave à l'eau, sèche et concentre à sec. On dissout le résidu dans 30 cm3 d'éthanol, ajoute 3 cm3 de lessive de soude, agite pendant 1 heure, dilue avec 90 cm3 d'eau et extrait au chloroforme, lave à

l'eau, sèche et concentre à sec. On recristallise le résidu dans le mélange chlorure de méthylène-éther isopropylique et recueille 1,21 g de produit attendu F = 204 ° C.

Stade B : γ-lactone de l'acide 17β-hydroxy 3-oxo 10β-(1-propynyl) 19-nor 17α-pregna 4,9 (11)-diène-21-carboxylique :

On ajoute en 15 minutes sous azote et à -50 ° C une solution de 3,6 cm3 de tétraméthylphosphorodiamidate d'allyle dans 15 cm3 de tétrahydrofuranne dans une solution de 23 cm3 de butyllithium à 1,6 M dans l'hexane et 20 cm3 de tétrahydrofuranne anhydre. On agite cette solution pendant une heure à -30 ° C et ajoute en 5 minutes 1,78 g de produit obtenu comme précédemment.
Après 2 heures 20,on introduit dans le mélange 38 cm3 d'acide chlorhydrique 2N,agite 30 minutes, extrait au chloroforme,lave à l'eau, sèche et concentre à sec. On dissout le résidu dans 30 cm3 d'éthanol, ajoute 6 cm3 d'acide chlorhydrique 6N, chauffe vers 50 ° C pendant 1 heure 15 minutes, distille l'éthanol, reprend par du chlorure de méthylène, lave à l'eau,sèche et concentre à sec. On chromatographie le résidu sur silice, élue par un mélange éther de pétrole (eb. : 60-80 ° C)-acétate d'éthyle (1-1) et obtient 920 mg de produit que l'on reprend par 2 cm3 de chlorure de méthylène, filtre, ajoute 10 cm3 d'éther isopropylique, concentre jusqu'à cristallisation, glace, essore et obtient après séchage 790 mg de produit attendu F = 200 ° C.
$/\alpha/_D$ : + 32 ° (c = 1 %, dans $CHCl_3$)

Exemple 5 : γ-lactone de l'acide 17β-hydroxy 3-oxo 100β-(phényléthynyl) 19-nor 17α-pregna 4,9 (11)-diène-21-carboxylique :

Stade A : 3,3-éthylène dioxy 5α-hydroxy 10β-(phényléthynyl) 17α-triméthylsilyloxy estr 9 (11)-ène 17β-carbonitrile :

Dans 50 cm3 d'une solution éthérée de bromure d'éthylmagnésium (M) on ajoute goutte à goutte 8 cm3 de phénylacétylène, dilue avec 35 cm3 de tétrahydrofuranne anhydre et laisse 1 heure à température ambiante. On ajoute, alors 6,45 g de 5α, 10α-époxy 3-oxo 17α-triméthylsilyloxy estr-9(11)ène 17β-carbonitrile,agite pendant 4 heures à température ambiante,chauffe 1 heure à 35 ° C. On refroidit et verse le mélange réactionnel dans une solution de chlorure d'ammonium, extrait à l'acétate d'éthyle, lave à l'eau, sèche, concentre à sec et obtient 8,9 g de produit. On le chromatographie sur silice en éluant par un mélange cyclohexane-acétate d'éthyle (9-1) et recueille 4,6 g de produit que l'on recristallise dans l'hexane et on obtient 3,47 g de produit attendu F = 176 ° C.

Stade B : 3,3-éthylène dioxy 5α-hydroxy 10β-(phényléthynyl) estr 9(11)-ène 17-one :

On agite 3,1 g de produit obtenu ci-dessus dans 31 cm3 d'éthanol, ajoute 3,1 cm3 de lessive de soude puis 10 cm3 d'éthanol et poursuit l'agitation pendant 2 heures. On dilue avec 100 cm3 d'eau, essore, lave à l'eau, sèche et obtient 2,49 g de produit attendu F = 199 ° C.

Stade C : γ-lactone de l'acide 17β-hydroxy 3-oxo 10β-(phényléthynyl) 19-nor 17α-pregna 4,9 (11)diène-21-carboxylique :

On refroidit à -70 ° C, 10 cm3 d'une solution 1,6 M de n-butyllithium dans l'hexane, dilue avec 10 cm3 de tétrahydrofuranne anhydre puis ajoute goutte à goutte à -70 ° C 1,6 cm3 de N,N,N',N'-tétraméthylphosphorodiamidate d'allyle dans 10 cm3 de tétrahydrofuranne et laisse réagir pendant 1 heure à -10 ° C.

On ajoute 865 mg du produit obtenu ci-dessus,agite pendant 15 heures à température ambianté. On acidifie en ajoutant 10 cm3 d'acide chlorhydrique 2N et 1 cm3 d'acide chlorhydrique concentré et agite pendant 30 minutes. On extrait à l'acétate d'éthyle, lave à l'eau la phase organique, sèche et concentre à sec sous pression réduite. On ajoute 25 cm3 de méthanol au résidu, puis 5 cm3 d'acide chlorhydrique 6 N chauffe à 50 ° C pendant 1 heure. Après refroidissement on dilue à l'eau et extrait à l'acétate d'éthyle, lave à l'eau la phase organique,sèche et concentre à sec sous pression réduite. On chromatographie le résidu sur silice en éluant par un mélange cyclohexane-acétate d'éthyle (1-1) et recueille 540 mg de produit attendu.

Après 2 cristallisations dans l'éther isopropylique puis l'éthanol aqueux le produit fond à 196 ° C.

Exemple 6 : γ-lactone de l'acide 17β-hydroxy 10β-/3-hydroxy prop-1-ynyl/ 3-oxo 19-nor 17α-pregna 4,9

(11)-diène-21-carboxylique :

Stade A : 3,3-éthylène dioxy 5α-hydroxy 10β-/3-(2RS-tétrahydropyrannyloxy) prop-1-ynyl/ méthylsilyloxy estr-9(11)-ène 17β-carbonitrile :

Dans 30 cm3 d'une solution éthérée de chlorure de propylmagnésium 0,9 M on ajoute goutte à goutte 4,4 cm3 de 3-(2-tétrahydropyrannyloxy) 1-propyne dans 15 cm3 d'éther anhydre. On dilue avec 20 cm3 de tétrahydrofuranne et agite pendant 50 minutes à température ambiante. On introduit 1,32 g de 3,3-éthylène dioxy 5α, 10α-èpoxy 17α-triméthylsilyloxy estr-9 (11)ène-17β-carbonitrile et agite pendant 5 heures à température ambiante, chauffe 1 heure à 35°C puis verse dans une solution diluée de phosphate monosodique. On extrait avec de l'acétate d'éthyle, lave à l'eau, sèche et concentre à sec. On chromatographie le résidu sur silice, élue par un mélange éther de pétrole-acétate d'éthyle (4-1) et obtient 1,335 g de produit attendu.

Stade B : 3,3-éthylène dioxy 5α-hydroxy 10β-/3(2RS-tétrahydropyrannyloxy) prop-1-ynyl/ estr 9(11)ène 17-one :

1,330 g de produit obtenu ci-dessus sont dissous dans 60 cm3 de méthanol. On ajoute 3 cm3 de lessive de soude et laisse réagir pendant 2 heures. On dilue avec 200 cm3 d'eau, amorce la cristallisation, essore, lave à l'eau, sèche et obtient 900 mg de produit attendu F = 143°C.

Stade C : γ-lactone de l'acide 5α, 17β-dihydroxy 3,3-éthylène dioxy 10β-/3-(2RS-tétrahydropyrannyloxy) prop-1-ynyl/ 19-nor 17α-pregna 9 (11)-ène-21-carboxylique :

A une solution de 12,5 cm3 de butyllithiun dans l'hexane à 1,6 M et refroidie à -50°C,on ajoute 12,5 cm3 de tétrahydrofuranne anhydre puis ajoute à -65°C une solution de 2,2 cm3 de tétraméthylphosphorotriamidate d'allyle dans 8 cm3 de tétrahydrofuranne sec. On amène à -10°,-15°C et laisse agir pendant une heure puis ajoute 890mg de produit obtenu ci-dessuset laisse réagir pendant 18 heures à température ambiante. On dilue à l'eau, extrait à l'acétate d'éthyle, lave à l'eau, sèche et concentre à sec. On chromatographie le résidu sur silice en éluant par un mélange benzène-acétate d'éthyle (7-3). On recueille 560 mg de produit attendu.

Stade D : γ-lactone de l'acide 17β-hydroxy 10β-/3-hydroxy prop-1-ynyl/ 3-oxo 19-nor 17α-pregna 4,9 (11)-diène-21-carboxylique :

On dissout 555 mg du produit obtenu ci-dessus dans 10 cm3 de méthanol avec 5 cm3 d'acide chlorhydrique 6 N et laisse réagir pendant 18 heures à température ambiante puis 1 heure à 60°C. On refroidit, dilue à l'eau, extrait au chlorure de méthylène,lave à l'eau, sèche et concentre à sec sous pression réduite. On chromatographie le résidu sur silice élue avec un mélange cyclohexane-acétate d'éthyle (1-1). On sépare 350 mg de produit attendu.

Après recristallisation dans l'acétate d'éthyle le produit fond à 198°C.

$/\alpha/_D$ = +31° ± 1 (c = 1 %, $CHCl_3$).

Exemple 7 : γ-lactone de l'acide 10β-éthynyl 17β-hydroxy 3-oxo 19-nor 17α-pregna 4, 6, 9 (11)-triène 21-carboxylique :

Stade A : γ-lactone de l'acide 3 éthoxy-10β-éthynyl 17β-hydroxy 19-nor 17α-pregna 3, 5, 9 (11)-triène 21-carboxylique :

A une solution de 6,77 g de produit obtenu à l'exemple 1 dans 68 cm3 de dioxanne, on ajoute sous agitation et azote 20 cm3 d'orthoformiate d'éthyle puis 203 mg d'acide paratoluènesulfonique monohydraté. Après 2 heures 30 on ajoute 0,6 cm3 de triéthylamine puis verse dans 60 cm3 de solution aqueuse saturée de bicarbonate de sodium. On extrait le produit attendu au chlorure de méthylène et obtient 12,35 g de produit brut que l'on chromatographie sur silice (éluant : cyclohexane-acétate d'éthyle (7-3), triéthylamine 1°/°°). On obtient 6,1 g de produit attendu F = 196°C.

Stade B : γ-lactone de l'acide 10β-éthynyl 17β-hydroxy 3-oxo 19-nor 17-pregna 4, 6, 9 (11)-triène 21-carboxylique :

A une solution de 2,164 g de produit obtenu au stade A dans 43 cm3 d'acétone à 5 % d'eau,on ajoute sous agitation 2,164 g de chloranile. On agite 22 heures à température ambiante, précipite le mélange réactionnel dans 100 cm3 d'un mélange eau saturée de bicarbonate de sodium-eau à 10 % de thiosulfate de sodium (1-1). On extrait au chlorure de méthylène, lave à l'eau, sèche et amène à sec. On obtient 2,1 g de produit brut que l'on chromatographie sur silice (éluant chlorure de méthylène-acétone 4-1) et isole 1,947 g de produit cristallisé que l'on empâte dans 10 cm3 d'éther éthylique. Après essorage, lavage à l'éther éthylique, séchage sous vide on isole 1,847 g de produit que l'on dissout dans un mélange éthanol-chlorure de méthylène, on chasse le chlorure de méthylène, glace, essore, lave à l'éthanol et sèche. On obtient 1,751 g de produit.

$/\alpha/_D$ = 243,5° ± 4,5° (c = 0,5 %, $CHCl_3$)

| Analyse : $C_{23}H_{24}O_3$ | | |
|---|---|---|
| Calculé : | C % : 79,28 | H % : 6,94 |
| Trouvé : | 79,2 | 7,0 |

Exemple 8 : γ-lactone de l'acide 7α-(acétylthio) 10β-éthynyl 17β-hydroxy 3-oxo 19-nor 17α-pregna 4,9 (11)-diène 21-caboxylique :

On agite 24 heures à température ambiante sous azote un mélange de 1,7 g de produit obtenu à l'exemple 7, 85 cm3 d'acide acétique, 3,4 cm3 d'acide chlorhydrique concentré et 0,85 cm3 d'acide thioacétique. On verse dans une solution aqueuse saturée de bicarbonate de sodium, extrait à l'acétate d'éthyle,lave à l'eau et obtient 2 g de résine que l'on chromatographie sur silice (éluant : cyclohexaneacétate d'éthyle 3-2) et isole 753 mg de produit attendu amorphe.

| Analyse : $C_{25}H_{28}O_5S$ | | | |
|---|---|---|---|
| Calculé : | C % : 70,72 | H % : 6,64 | S % : 7,55 |
| Trouvé : | 70,5 | 6,7 | 7,3 |

Exemple 9 : γ-lactone de l'acide 10β-éthynyl 17β-hydroxy 3-oxo 7α-propyl 19-nor 17α-pregna 4,9 (11)-diène-21-carboxylique :

a) Préparation du bromure de propylmagnésium :

A un mélange sous azote de 900 mg de magnésium et de 27 cm3 de tétrahydrofuranne on ajoute en 30 minutes à 30°C une solution de 2,7 cm3 de bromure de n-propyle dans 2,7 cm3 de tétrahydrofuranne. On agite une nuit à température ambiante sous azote.

b) Addtion du magnésien :

A 14 cm3 du magnésien précédent agité sous azote à -40°C on ajoute en 30 minutes la solution suivante : 1,514 g d'iodure cuivreux 0,69 g de bromure de lithium dans 3,7 cm3 de tétrahydrofuranne et on rince avec 1 cm3 de tétrahydrofuranne. On agite 15 minutes à -40°C la suspension obtenue. On refroidit à -70°C et ajoute en 10 minutes 1,05 cm3 d'éthérate de trifluorure de bore. On agite 20 minutes à -70°C et ajoute en 35 minutes 923 mg de γ-lactone de l'acide 10β-éthynyl 17β-hydroxy 3-oxo 19-nor 17α pregna 4, 6, 9 (11)-triène 21-carboxylique préparé à l'exemple 7 en solution dans 50 cm3 de tétrahydrofuranne. On rince avec 2 fois 5 cm3 de tétrahydrofuranne, agite à -70°C. Après 45 minutes on ajoute 4 cm3 d'acide chlorhydrique 5N. On laisse remonter à température ambiante puis agite 3 heures. On dilue à l'eau saturée de chlorure de sodium. On extrait à l'acétate d'éthyle, lave la phase organique à l'ammoniaque dilué puis à l'eau distillée, sèche et amène à sec sous vide et chromatographie le résidu sur silice (cyclohexaneacétate d'éthyle 7-3). On isole 480 mg de produit attendu. On dissout le produit dans le mélange chlorure de méthylène-éther isopropylique, distille le chlorure de méthylène et isole 460 mg de produit attendu. F = 148°C. $/\alpha/_D$ = +40° ± 1° (c =O,8 % dans l'éthanol)

| Analyse : $C_{26}H_{32}O_3$ = 392,5 | | |
|---|---|---|
| Calculé<br>Trouvé | C % : 79,55<br>: 79,6 | H % : 8,21<br>8,3 |

Lors de la chromatographie on isole également 247 mg de l'isomère 7$\beta$ le moins mobile que l'on recristallise de la même façon dans le mélange chlorure de méthylène-éther isopropylique et obtient 121 mg de produit F = 147° C.

$/\alpha/_D$ = -14° ± 2° (c = 0,5 % dans l'éthanol).

Exemple 10 : $\gamma$-lactone de l'acide 17$\beta$-hydroxy 3-oxo 10$\beta$-(1-propynyl) 19-nor 17$\alpha$-pregna 4, 6, 9 (11)-triène 21-carboxylique :

Stade A : $\gamma$-lactone de l'acide 3-éthoxy 17$\beta$-hydroxy 10$\beta$-(1-propynyl) 19-nor 17$\alpha$-pregna 3, 5, 9 (11)-triène 21-carboxylique :

On agite à température ambiante pendant 4 heures un mélange de 20,3 g de $\gamma$-lactone de l'acide 17$\beta$-hydroxy 3-oxo 10$\beta$-(1-propynyl) 19-nor 17$\alpha$-pregna 4,9(11)diène-21-carboxylique préparé à l'exemple 7 dans 200 cm3 de dioxanne, 60 cm3 d'orthoformiate d'éthyle et 0,6 g d'acide paratoluènesulfonique monohydraté. On ajoute 2 cm3 de triéthylamine puis verse lentement dans 200 cm3 de solution de carbonate acide de sodium. On extrait le précipité par du dichlorométhane. On obtient 31,1 g de produit que l'on dissout dans 100 cm3 de dichlorométhane. A cette solution on ajoute lentement 250 cm3 d'éther isopropylique puis glace. On obtient 5,8 g de cristaux F = 210° C. Les liqueurs-mères sont concentrées et le résidu est purifié par chromatographie (éluant cyclohexane-acétate d'éthyle 7-3). On récupère ainsi 13,2 mg de produit identique F = 210° C.

Stade B : $\gamma$-lactone de l'acide 17$\beta$-hydroxy 3-oxo 10$\beta$-(1-propynyl) 19-nor 17$\alpha$-pregna 4, 6, 9 (11)-triène 21-carboxylique :

On agite à température ambiante pendant 22 heures le mélange de 5,8 g de produit obtenu au stade A, 116 cm3 d'acétone à 5 % d'eau et 5,8 g de chloranile. On verse dans un mélange de 150 g d'une solution à 10 % de carbonate acide de sodium dans l'eau et de 150 g de solution de thiosulfate de sodium.

On extrait avec du dichlorométhane. On chasse les solvants et obtient 6,9 g de produit que l'on purifie par chromatographie sur silice (éluant acétate d'éthyle-éther de pétrole 1-1). On obtient 5,1 g de produit que l'on recristallise par dissolution dans 20 cm3 de dichlorométhane et addition de 200 cm3 d'oxyde d'isopropyle. On obtient ainsi 4,77 g de cristaux. F = 248° C.

Exemple 11 : $\gamma$-lactone de l'acide 10$\beta$-(1-butynyl) 17$\beta$-hydroxy 3-oxo 19-nor 17$\alpha$-pregna-4,9 (11)diène 21-carboxylique :

Stade A : 10$\beta$-(1-butynyl) 3,3-(éthylène dioxy) 5$\alpha$-hydroxy estr-9(11)ène 17-one :

On fait barboter pendant 2 heures 30 de l'éthyl acétylène dans un mélange de 330 cm3 de bromure d'éthylmagnésium en solution éthérée 0,5 N et 330 cm3 de tétrahydrofuranne puis introduit en une seule fois 10 g de 3,3-éthylène dioxy 5$\alpha$, 10$\alpha$-époxy 17$\alpha$-triméthylsilyloxy estr-9 (11)ène 17$\beta$-carbonitrile. On chauffe à 40-50° C pendant 4 heures 30, ramène à température ambiante, verse dans un mélange chlorure d'ammonium-glace, décante, lave à l'eau, extrait au chloroforme, sèche puis amène à sec. Les 12 g de produit obtenus sont dissous dans 100 cm3 d'éthanol à 95° C, on ajoute 10 cm3 de lessive de soude, agite 45 minutes, distille l'éthanol, reprend au chloroforme, lave à l'eau, sèche puis amène à sec. Les 9,3 g de produit ainsi obtenus sont chromatographiés sur silice (éluant : éther de pétrole-acétate d'éthyle (1-1) à 1°/₀₀ de trièthylamine). On recueille 4,6 g de produit Rf. = 0,35. Ce produit est dissous dans 10 cm3 de chlorure de méthylène. On filtre, ajoute 30 cm3 d'éther isopropylique, glace puis essore et sèche à 50° C. On obtient 240 mg de produit F = 139° C.

| Analyse $C_{24}H_{32}O_4$ = 384,52 | | |
|---|---|---|
| Calculé<br>Trouvé | C % : 74,97<br>: 75,0 | H % : 8,39<br>8,5 |

Stade B : γ-lactone de l'acide 10β-(1-butynyl) 17β-hydroxy 3-oxo 19-nor 17 α-pregna-4,9 (11)-diène 21-carboxylique :

On ajoute à -50 ° C, 24 cm3 de tétrahydrofuranne à 24 cm3 de n-butyllithium en solution dans l'hexane à 1,65 M puis ajoute en 15 minutes à -50 ° C, 4 cm3 de téraméthylphosphorodiamidate d'allyle en solution dans 16 cm3 de tétrahydrofuranne. On agite pendant 1 heure à -30 ° C et introduit 1,92 g de produit obtenu au stade A en solution dans 20 cm3 de tétrahydrofuranne.

On laisse refroidir à température ambiante, agite pendant 2 heures, ajoute entre 0 et +5 ° C 40 cm3 d'acide chlorhydrique 2 N,agite 30 minutes, extrait au chloroforme, lave à l'eau,sèche et amène à sec. On obtient 3 g de produit que l'on dissout dans 30 cm3 d'éthanol, ajoute sous azote 6 cm3 d'acide chlorhydrique au demi et chauffe à 50 ° C pendant 2 heures. On amène à sec, reprend au chlorure de méthylène, lave à l'eau puis amène à sec. On obtient 2 g de produit que l'on chromatographie sur silice en éluant par de l'éther de pétrole-acétate d'éthyle 1-1. On obtient 1,26 g de produit Rf. = 0,22. Le produit est dissous dans 20 cm3 d'éther isopropylique au reflux. On concentre au demi, amorce la cristallisation, glace, essore, lave à l'éther isopropylique glacé puis sèche à 50 ° C sous vide. On obtient 880 mg de produit que l'on recristallise en le dissolvant dans 8 cm3 d'éther isopropylique au reflux, filtre, concentre jusqu'à début de trouble, amorce à chaud, glace, essore, lave à l'éther de pétrole puis sèche à 50 ° C sous vide. On obtient 770 mg de produit. F = 94 ° C.

| Analyse : $C_{25}H_{30}O_3$ = 378,48 | | |
|---|---|---|
| Calculé<br>Trouvé | C % : 79,33<br>: 79,5 | H % : 7,99<br>8,2 |

$/\alpha/_D$ = +35 ° ± 1 ° (c = 1 %, chloroforme)

Exemple 12 : γ-lactone de l'acide 17β-hydroxy 3-oxo 10β-(1-pentynyl) 19-nor 17α-pregna-4,9 (11)-diène 21-carboxylique :

Stade A : 3,3-(éthylènedioxy) 5α-hydroxy 10β-(1-pentynyl) estr 9 (11)-ène-17-one :

On opère comme au stade A de l'exemple 11 à partir de 6,45 g de 3,3-éthylène dioxy 5α, 10α-époxy 17α-triméthylsilyloxy estr-9 (11) ène 17β-carbonitrile et obtient après chromatographie 2,61 g de produit attendu. Une recristallisation de 200 mg de ce produit dans 2 cm3 d'éther isopropylique au reflux donne 140 mg de produit attendu pur F = 120 ° C.

| Analyse : $C_{25}H_{35}O_4$ = 398,55 | | |
|---|---|---|
| Calculé<br>Trouvé | C % : 75,34<br>: 75,1 | H % : 8,6<br>8,7 |

Stade B : γ-lactone de l'acide 17β-hydroxy 3-oxo 10β-(1-pentynyl) 19-nor 17α-pregna-4,9 (11)-diène 21-carboxylique :

On opère comme au stade B de l'exemple 11 à partir de 2 g de produit obtenu au stade A. Après chromatographie, on obtient 1,13 g de produit attendu. Ce produit est recristallisé par dissolution dans 2 cm3 de chlorure de méthylène et addition de 10 cm3 d'éther isopropylique. On obtient 970 mg de produit pur puis 910 mg lors d'une seconde recristallisation. F = 123 ° C.

| Analyse : $C_{26}H_{32}O_3$ = 392,52 | | |
|---|---|---|
| Calculé<br>Trouvé | C % : 79,55<br>: 79,6 | H % : 8,22<br>8,3 |

/α/$_D$ = +41° ± 1° (c = 1 %, chloroforme)

Exemple 13 : 17β-hydroxy 10β-(but-1-ynyl estr 4,9 (11) diène-3-one :

a) Réduction de la cétone en 17 :

On ajoute 385 mg de borohydrure de sodium à une solution de 770 mg de 10β-(but-1-ynyl) 3,3-(éthylène dioxy) 5α-hydroxy estr 9 (11)-ène 17-one préparé au stade A de l'exemple 11 dans 15 cm3 d'éthanol. On agite 45 minutes, distille à sec sous vide, reprend au chlorure de méthylène, lave à l'eau, sèche puis amène à sec. On obtient 770 mg de produit. F = 195°C.

b) Hydrolyse acide du cétal :

Les 770 mg de produit obtenus ci-desus sont dissous sous azote à 50-60°C dans 15 cm3 d'éthanol. On ajoute 0,8 cm3 d'acide chlorhydrique au demi. On chauffe pendant 4 heures, amène à sec reprend au chlorure de méthylène, lave à l'eau,sèche puis amène à sec. On obtient 750 mg de résine que l'on chromatographie sur silice (éluant éther de pétrole-acétate d'éthyle 1-1) Rf. = 0,33. On obtient 630 mg de produit que l'on recristallise en le dissolvant dans 6 cm3 d'éther isopropylique au reflux, puis on filtre, concentre a moitié, amorce, glace, essore, lave à l'éther isopropylique glacé puis sèche. On obtient 485 mg de produit. F = 95°C.
/α/$_D$ = +136° ± 2° (c = 1 %, chloroforme).

Exemple 14 : 17β-hydroxy 10β-(pent-1-ynyl estr 4,9 (11) diène-3-one :

On opère comme indiqué à l'exemple 13 à partir de 570 mg de 10β -(pent-1-ynyl 3,3 (éthylène dioxy) 5α-hydroxy estra 9 (11) ène 17-one préparé au stade A de l'exemple 12. On obtient 280 mg de produit attendu.
/α/$_D$ = +134° ± 2° (c = 1 %, chloroforme)

Exemple 15 : 17β-hydroxy 10β-(prop-1-ynyl) estr 4,9 (11) diène 3-one :

On opère comme indiqué à l'exemple 13 à partir de 520 mg de 10β (prop-1-ynyl) 3,3 (éthylène dioxy 5α-hydroxy estr-9 (11) ène 17-one préparé au stade A de l'exemple 4. On obtient 260 mg de produit attendu F = 154°C.
/α/$_D$ = 130° ± 1° (c = 1 % chloroforme)

Exemple 16 : γ-lactone de l'acide 17β-hydroxy 3-oxo 7α-propyl 10β (1-propynyl) 19-nor 17α-pregna 4,9 (11) diène 21-carboxylique :

En opérant comme à l'exemple 9 au départ du produit de l'exemple 10, on obtient le produit attendu . F = 110°C Rf. = 0,2 (éluant cyclohexane-acétate d'éthyle : 7-3).

| Analyse : $C_{27}H_{34}O_5$ | | |
|---|---|---|
| Calculé<br>Trouvé | C % : 79,77<br>: 76,6 | H % : 8,43<br>8,5 |

Exemple 17 : γ-lactone de l'acide 7α-acétylthio 17β-hydroxy 3-oxo 10β-(1-propynyl) 19-nor 17α-pregna 4,9 (11) diène 21-carboxylique :

En opérant comme à l'exemple 8 au départ du produit de l'exemple 10, on obtient le produit attendu.

Rf. = 0,1 (éluant : dichlorométhane-acétone 9-2).

| Analyse : $C_{26}H_{30}SO_4$ = 458,59 | | | |
|---|---|---|---|
| Calculé<br>Trouvé | C % : 71,20<br>: 71 | H % : 6,89<br>6,8 | S % : 7,31<br>7,2 |

Exemple 18 : 10β-(3-hydroxy 1-propynyl) 17β-hydroxy estra 4,9 (11) dièn-3-one.

a) Réduction de la cétone en 17.

On ajoute 58 mg de borohydrure de sodium à une solution refroidie à +5°C comprenant 290 mg de 3,3-éthylènedioxy 5α-hydroxy 10β-/3-(2RS-tétrahydropyrannyloxy) 1-propynyl/ estr-9 (11)-èn-17-one préparé comme à l'exemple 6 stade B et 6 cm3 de méthanol. On laisse 2 heures sous agitation, dilue à l'eau, extrait au chlorure de méthylène, concentre à sec et obtient 300 mg de produit.

b) Hydrolyse acide du cétal.

On reprend le produit ci-dessus dans 6 cm3 de méthanol, ajoute 3 cm3 d'acide chlorhydrique au demi, agite pendant 1 heure, dilue à l'eau, amorce la crsitallisation, essore, sèche et obtient 166 mg de produit attendu. F = 204°C après recristallisation dans un mélange chlorure de méthylène-éther isopropylique. $/\alpha/_D$ = +124° (c = 1%, chloroforme).

Exemple 19 : γ-lactone de l'acide 17β -hydroxy 3-oxo 10β(1-propynyl) 19-nor 17α-pregna 1,4,9 (11) trièn-21-carboxylique.

On chauffe 20 heures au reflux 2 g de produit obtenu à l'exemple 4 en suspension dans 20 cm3 de toluène, en présence de 3,2 g d'anhydride phényl séléninique, laisse refroidir, verse sur une solution de bicarbonate de sodium, extrait à l'acétate d'éthyle, lave avec une solution aqueuse d'hydroxyde de sodium, sèche et évapore les solvants sous pression réduite. Après chromatographie sur silice (éluant : chlorure de méthylène-acétone 97-3), on obtient 0,511 g de produit attendu.

Spectre IR (chloroforme)

1764 $cm^{-1}$ : γ-lactone ; 1667 $cm^{-1}$ : cétone conjuguée :
1631-1608 $cm^{-1}$ : -C≡C-.

Exemple 20 : γ-lactone de l'acide 17β-hydroxy 7α-méthyl 3-oxo 10β-(1-propynyl) 19-nor 17α-pregna 4,9 (11)-dièn-21-carboxylique.

On mélange 75 mg de chlorure cuivrique et 24 mg de chlorure de lithium dans 55 cm3 de tétrahydrofuranne et ajoute sous atmosphère inerte 2 g de produit obtenu à l'exemple 10. On refroidit à +2°/+4°C et ajoute goutte à goutte en 2 heures 7,9 cm3 d'une solution éthérée d'iodure de méthyl magnésium 1,05M, agite 2 heures en maintenant la température à +2°C, ajoute ( cm3 d'acide chlorhdyrique 5N et laisse revenir à température ambiante. On extrait le milieu réactionnel à l'acétate d'éthyle, lave à l'eau, puis avec une solution aqueuse d'hydroxyde d'ammonium, puis à l'eau, sèche et concentre à sec. Après chromatographie sur silice (éluant : cyclohexane-acétate d'éthyle 8,5-1,5), on obtient 384 mg d'isomère 7α , F = 125°C et 550 mg d'isomère 7β du produit attendu.

| Analyse : $C_{25}H_{30}O_3$ : 378,52 | | | |
|---|---|---|---|
| isomère<br>7α : | Calculé :<br>Trouvé : | C% 79,33<br>79,0 | H% 7,99<br>8,1 |
| isomère<br>7β : | Calculé :<br>Trouvé : | C% 79,33<br>79,3 | H% 7,99<br>8,0 |

Exemple 21 : γ-lactone de l'acide (Z) 17β -hydroxy 3-oxo 10β-(1-propényl) 19-nor 17α-pregna 4,9 (11)-dièn-21-carboxylique.

On ajoute 8 cm3 de pyridine puis 0,29 g de catalyseur de Lindlar à une solution de 1,45 g de produit obtenu à l'exemple 4 dans 32 cm3 d'acétate d'éthyle. On hydrogène pendant 2 heures, essore le catalyseur, élimine la pyridine par lavage à l'acide chlorhydrique 2N, décante la phase organique, la lave à l'eau, la sèche et la concentre à sec. On reprend le résidu dans l'éther, chauffe au reflux, refroidit, essore les cristaux, les sèche et obtient 840 mg de produit attendu. F = 170 °C après recristallisation dans un mélange chlorure de méthylène-éther isopropylique.
$/\alpha/_D$ = -47 ° (c = 1%, chloroforme).

| Analyse : $C_{24}H_{30}O_3$ : 366,51 | | |
|---|---|---|
| Calculé : | C% 78,65 | H % 8,25 |
| Trouvé : | 78,4 | 8,4 |

Exemple 22 : γ-lactone de l'acide 10β -(3-chloro 1-propynyl) 17β-hydroxy 3-oxo 19-nor 17α-pregna 4,9 (11)-dièn-21-carboxylique.

On ajoute 1 g de triphénylphosphine à une solution de 0,5 g de produit obtenu à l'exemple 6 dans 5 cm3 de tétrahydrofuranne et 5 cm3 de tétrachlorure de carbone. On chauffe 30 minutes à 80 °C sous agitation, concentre à sec sous pression réduite, chromatographie le résidu sur silice (éluant : cyclohexane-acétate d'éthyle 9-1 puis 7-3). On purifie le produit par chromatographie sur silice en éluant à l'éther et obtient 0,38 g de produit attendu. F = 134 °C après recristallisation dans un mélange chlorure de méthylène-éther isopropylique.
$/\alpha/_D$ = +17,5 ° (c = 1%, chloroforme).

| Analyse : $C_{24}H_{27}O_3Cl$ : 398,93 | | | |
|---|---|---|---|
| Calculé : | C% 72,26 | H % 6,82 | Cl% 8,88 |
| Trouvé : | 72,1 | 6,8 | 9,2 |

Exemple 23 : (6α,7α,17α)γ-lactone de l'acide 6,7-dihydro 17β-hydroxy 3-oxo 10β-(1-propynyl) 3'H-cyclopropa-(6,7)-19-nor pregna 4,9 (11)-dièn-21-carboxylique.

Dans 4,04 g d'iodure de triméthyl sulfoxonium en solution dans 15 cm3 de diméthylsulfoxyde, on ajoute sous atmosphère inerte 8,6 cm3 de terbutylate de potassium en solution dans 8,2 cm3 de tétrahydrofuranne, agite 15 minutes, ajoute 2,737 g de produit obtenu comme à l'exemple 10 dans 3 cm3 de tétrahydrofuranne et chauffe à 59 °/60 °C pendant 1 heure. On glace, ajoute 8 cm3 d'acide chlorhydrique N, dilue avec une solution aqueuse saturée de chlorure de sodium, extrait à l'acétate d'éthyle, lave la phase organique avec une solution aqueuse saturée de bicarbonate de sodium, puis avec une solution aqueuse saturée de chlorure de sodium. On sèche, concentre à sec, purifie par chromatographie sur silice (éluant : cyclohexane-acétate d'éthyle 6-4) et obtient 1,28 g de produit attendu. F = 243 °C après recristallisation dans l'isopropanol.
$/\alpha/_D$ =-276 ° (c = 0,5%, $CHCl_3$).

| Analyse : | | |
|---|---|---|
| Calculé : | C% 79,75 | H % 7,49 |
| Trouvé : | 79,4 | 7,6 |

Exemple 24 : 13β-éthyl 10β-éthynyl 17β-hydroxy gona 4,9 (11)-dièn-3-one.

Stade A : 13β-éthyl 3,3-éthylènedioxy 10β-éthynyl gon-9 (11)-ène 5α, 17β-diol.

On dissout 3,9 g de 3,3-éthylènedioxy 5α,10α-époxy, 13β-éthyl 17β-hydroxy gon-9 (11)-ène préparé comme dans le brevet français 2 377 417 dans du benzène, concentre sous pression réduite et reprend le résidu dans 40 cm3 d'éthylène diamine. On ajoute sous atmosphère inerte 3,6 g de complexe acétylure de lithium-éthylène diamine, chauffe à 45 ° C et maintient 24 heures sous agitation, ajoute de nouveau 3,9 g de complexe, agite 72 heures à 48 ° C, refroidit et verse dans une solution de chlorure d'ammonium. On extrait à l'acétate d'éthyle, lave la phase organique à l'eau, la sèche et chasse les solvants sous pression réduite. On chromatographie le résidu sur silice (éluant : cyclohexane-acétate d'éthyle 1-1) et obtient 2,3 g de produit attendu.

Stade B : 13β-éthyl 10β-éthynyl 17β-hydroxy gona 4,9 (11)-dièn-3-one.

On ajoute 3,5 cm3 d'acide chlorhydrique au demi à 0,7 g de produit obtenu au stade A dans 7 cm3 de méthanol. On chauffe à 55 ° C pendant 1 heure, dilue à l'eau et extrait au chlorure de méthylène. On lave la phase organique à l'eau, sèche, concentre à sec sous pression réduite, chromatographie le résidu sur silice (éluant : cyclohexane-acétate d'éthyle (1-1), purifie le produit obtenu par une nouvelle chromatographie sur silice (éluant : éther-hexane 6-4) et recueille 0,39 g de produit attendu. F = 120 ° C après recristallisation dans l'éther isopropylique.
$/\alpha/_D$ = +98,5 ° (c = 1%, chloroforme).

| Analyse : $C_{21}H_{26}O_2$ : 310,44 | | |
|---|---|---|
| Calculé : | C% 81,25 | H% 8,44 |
| Trouvé : | 81,1 | 8,7 |

Exemple 25 : γ-lactone de l'acide 13β-éthyl 10β-éthynyl 17β -hydroxy 3-oxo 18,19-dinor (17α)-pregna-4,9 (11) dièn-21-carboxylique.

Stade A : 17β-éthyl 10β-éthynyl 3,3-éthylènedioxy 5α-hydroxy gon-9 (11)-èn-17-one.

On ajoute 2,4 g de dichromate de pyridinium dans une solution refroidie à +4 ° C de 0,75 g de produit obtenu au stade A de l'exemple 24 dans 12 cm3 de diméthylformamide. On agite 1 heure 30 minutes, dilue à l'eau et extrait à l'éther. On lave la phase organique à l'eau, la sèche et la concentre à sec. On obtient 0,74 g de produit attendu.

Stade B : γ-lactone de l'acide 13β-éthyl 10β-éthynyl 17β-hydroxy 3-oxo 18,19-dinor (17α)-pregna-4,9 (11) dièn-21-carboxylique.

On opère comme au stade B de l'exemple 4 à partir de 0,95 g de produit obtenu au stade A précédent et en laissant réagir pendant 65 heures à température ambiante. On verse le milieu réactionnel dans une solution de chlorure d'ammonium et extrait à l'acétate d'éthyle. On lave la phase organique à l'eau, la sèche et la concentre à sec sous pression réduite. On reprend le résidu dans 18 cm3 de méthanol , ajoute 9 cm3 d'acide chlorhydrique au demi, chauffe 1 heure 30 minutes à 50 °/55 ° C, refroidit, dilue à l'eau, extrait au chlorure de méthylène, purifie le produit brut obtenu par chromatographie sur silice (éluant : éther-acétate d'éthyle 8,5-1,5) et obtient 0,26 g de produit attendu. F = 198 ° C après recristallisation dans l'éther isopropylique.
$/\alpha/_D$ = +4 ° (c = 1%, chloroforme).

| Analyse : $C_{24}H_{28}O_3$ : 364,49 | | |
|---|---|---|
| Calculé : | C% 79,08 | H% 7,74 |
| Trouvé : | 79,1 | 7,8 |

Exemple 26 : 10β-éthynyl 17β-hydroxy 17α-méthyl estra 4,9 (11) dièn-3-one.

Stade A : 3,3-éthylènedioxy 10β-éthynyl 5α,17β-dihydroxy 17α-méthyl estr-9 (11)-ène.

On ajoute en 15 minutes à -65°C 27 cm3 d'une solution de méthyl lithium dans l'éther (1,8M) à une suspension de 3,05 g d'iodure de cuivre dans 30 cm3 de tétrahydrofuranne. On agite 15 minutes, ajoute 713 mg de 3,3-éthylènedioxy 10β-éthynyl 5α-hydroxy estra 9 (11)-èn-17-one en solution dans 20 cm3 de tétrahydrofuranne, maintient sous agitation 2 heures à -65°C et laisse revenir à température ambiante. On agite 39 heures, glace, ajoute une solution aqueuse saturée en chlorure d'ammonium, extrait à l'acétate d'éthyle, lave la phase organique à l'eau, sèche et concentre à sec. On chromatographie le résidu sur silice (éluant : cyclohexane-acétate d'éthyle 6-4) et obtient 560 mg de produit attendu.

Stade B : 10β-éthynyl 17β-hydroxy 17α-méthyl estra 4,9 (11) dièn-3-one.

On chauffe 3 heures 15 minutes sous atmosphère inerte 704 mg de produit préparé comme au stade A précédent, dans 14 cm3 d'éthanol et 3,5 cm3 d'acide chlorhydrique 5N. On glace, ajoute 2 m3 d'ammoniaque, chasse l'éthanol sous pression réduite, dilue à l'eau et extrait à l'acétate d'éthyle. On lave la phase organique à l'eau, sèche et concentre à sec. On chromatographie le résidu sur silice (éluant : cyclohexane-acétate d'éthyle 6-4) et obtient 472 mg de produit attendu. F = 174°C après cristallisation dans l'éther éthylique.
$/\alpha/_D$ = +65,5° ± 2° (c = 0,5%, chloroforme).

| Analyse : | | |
|---|---|---|
| Calculé : | C% 81,24 | H% 8,44 |
| Trouvé : | 81,2 | 8,3 |

Exemple 27 : 10β-éthynyl 17β-hydroxy 17α-éthyl estra 4,9 (11) dièn-3-one.

Stade A : 3,3-éthylènedioxy 10β-éthynyl 5α-,17β-dihydroxy 17α-éthyl estr 9 (11) ène.

On ajoute 100 mg de bromure cuivreux dans 40 cm3 d'une solution de bromure de méthyl magnésium (0,5M) refroidie à +10°C. On agite 20 minutes, ajoute 1 g de produit préparé à l'exemple 1 stade A et maintient l'agitation pendant 2 heures 30 minutes. On verse sur une solution aqueuse saturée en chlorure d'ammonium à 0°C, extrait au chlorure de méthylène, concentre à sec et obtient 1,04 g de produit attendu.

Stade B : 10β-éthynyl 17β-hydroxy 17α-éthyl estra 4,9 (11) dièn-3-one.

On agite 16 heures à température ambiante puis 3 heures à 40°C 971 mg de produit obtenu au stade A précédent dans 20 cm3 d'éthanol et 10 cm3 d'acide chlorhydrique 2H. On refroidit, neutralise à l'ammoniaque et extrait au chlorure de méthylène. On évapore le solvant, chromatographie le résidu sur silice (éluant : hexane-acétate d'éthyle 6-4) et obtient après cristallisation dans l'éther éthylique 372 mg de produit attendu. F = 147-148°C.
$/\alpha/_D$ = +56° + 5° (c = 0,2%, chloroforme).

Exemple 28 : 10β-éthynyl 17β-hydroxy 17α-propyl estra 4,9 (11) dièn-3-one.

Stade A : 3,3-éthylènedioxy 10β-éthynyl 5α, 17β-dihydroxy 17α-propyl estra 9 (11) ène.

On opère comme au stade A de l'exemple 27 à partir de 465 mg de bromure cuivreux, de 90 cm3 d'une solution tétrahydrofurannique de bromure d'éthyl d'ammonium (0,7M) et de 4,650 g de produit préparé comme au stade A de l'exemple 1. Après extraction à l'acétate d'éthyle et purification par chromatographie sur silice (éluant : cyclohexane-acétate d'éthyle), on obtient 4,375 g de produit attendu.

Stade B : 10β-éthynyl 17β-hydroxy 17α-propyl estra 4,9 (11) dièn-3-one.

On ajoute 21 cm3 d'acide chlorhydrique 5H dans une solution de 4,2 g de 3,3-éthylènedioxy 10β-éthynyl 5α, 17β-dihydroxy 17α-propyl estra 9 (11) ène obtenu au stade A précédent dans 130 cm3 d'éthanol et chauffe 2 heures 30 minutes à 55°C environ sous agitation et atmosphère inerte. On concentre partiellement, dilue à l'acétate d'éthyle, lave à l'eau, sèche et concentre à sec. On chromatographie le résidu sur silice (éluant : cyclohexane-acétate d'éthyle 8-2) et obtient 2,8 g de produit attendu. F = 167°C

après recristallisation dans l'isopropanol.
$/\alpha/_D$ = +58,5° + 2,5° (c = 0,5%, chloroforme).

Exemple 29 : 10β-éthynyl 17β-hydroxy 17α-butyl estra 4,9 (11) dièn-3-one.

Stade A : 3,3-éthylènedioxy 10β-éthynyl 5α, 17β-dihydroxy 17α-butyl estr 9 (11) ène.

On agite 30 minutes à +7°C 100 mg de bromure cuivreux dans 18 cm3 d'une solution tétrahydrofurannique de bromure de n-propyl magnésium et ajoute 1 g de produit préparé comme au stade A de l'exemple 1 en solution dans 10 cm3 de tétrahydrofuranne. On laisse revenir à température ambiante, agite 45 minutes et verse sur une solution aqueuse saturée en chlorure d'ammonium glacée, extrait à l'acétate d'éthyle, lave la phase organique à l'eau, puis avec une solution aqueuse saturée de chlorure de sodium, concentre à sec, purifie le résidu par chromatographie sur silice (éluant : cyclohexane-acétate d'éthyle 6-4 à 1°/₀₀ de triéthylamine). On obtient après cristallisation dans l'éther 1 g de produit attendu. F ≃ 130°C.

Stade B : 10β-éthynyl 17β-hydroxy 17α-butyl estra 4,9 (11) dièn-3-one.

On agite 6 heures à 40°C puis 16 heures à température ambiante 870 mg de produit obtenu au stade A dans 26 cm3 d'éthanol et 34 cm3 d'acide chlorhydrique 2N. On verse dans de l'eau glacée, alcalinise avec de l'ammoniaque concentrée, extrait à l'acétate d'éthyle, lave la phase organique à l'eau puis avec une solution aqueuse saturée de chlorure de sodium, sèche et concentre à sec. On chromatographie le résidu sur silice (éluant : cyclohexane-acétate d'éthyle 6-4) et obtient après recristallisation dans l'éther isopropylique 554 mg de produit attendu. F = 134°C.
$/\alpha/_D$ = +45,5° + 2° (c = 0,5%, chloroforme).

| Analyse : | | |
|---|---|---|
| Calculé : | C% 81,77 | H% 9,15 |
| Trouvé : | 82,0 | 9,2 |

Exemple 30 : 10β-éthynyl 17β-hydroxy 17α-(2-propén-1-yl) estra 4,9 (11) dièn-3-one.

Stade A : 3,3-éthylènedioxy 10β-éthynyl 5α, 17β-dihydroxy 17α-(2-propén-1-yl) estr 9 (11) ène.

On introduit en 10 minutes une solution comprenant 1 g de 3,3-éthylènedioxy 10β-éthynyl 5α-hydroxy estr 9 (11) èn-17-one et 8 cm3 de tétrahydrofuranne dans 15,6 cm3 d'une solution tétrahydrofurannique de chlorure d'allyle magnésium 0,9M et agite pendant 4 heures. On verse sur une solution aqueuse saturée en chlorure d'ammonium à 0°C, extrait au chlorure de méthylène, chasse le solvant, purifie par chromatographie sur silice (éluant : hexane-acétate d'éthyle 1-1 à 1% de triéthylamine) et obtient 987 mg de produit attendu.

Stade B : 10β-éthynyl 17β-hydroxy 17α-(2-propén-1-yl) estra 4,9 (11) dièn-3-one.

On agite 2 heures à température ambiante puis 3 heures à 50°C 940 mg de produit obtenu au stade A précédent dans 20 cm3 d'éthanol et 5 cm3 d'acide chlorhydrique 5N. On refroidit, neutralise à l'ammoniaque et extrait au chlorure de méthylène. On évapore le solvant, chromatographie le résidu sur silice (éluant : cyclohexane-acétate d'éthyle 7-3) et obtient après cristallisation dans l'éthanol 475 mg de produit attendu. F = 150°C.

| Analyse : $C_{23}H_{28}O_2$ : 336,477 | | |
|---|---|---|
| Calculé : | C% 81,9 | H% 8,60 |
| Trouvé : | 82,1 | 8,39 |

Exemple 31 : 10β-éthynyl 17β-hydroxy 17α-(3-hydroxy propyl) estra 4,9 (11) dièn-3-one.

Stade A : 3,3-éthylènedioxy 10β-éthynyl 5α, 17β-dihydroxy 17α-/3-(1,1-diméthyléthoxy) propyl/ estr 9 (11) ène.

1) Préparation du magnésien.

On chauffe à 60°C sous atmosphère inerte 16 g de magnésium dans 200 cm3 de tétrahydrofuranne. On ajoute quelques gouttes de 1,2-dibromoéthane puis en 30 minutes 49,7 g de 3-(1,1-diméthyléthoxy) chloropropane en solution dans 100 cm3 de tétrahydrofuranne et chauffe 3 heures au reflux.

2) Condensation.

On chauffe au reflux sous atmosphère inerte 64 cm3 de magnésien préparé ci-dessus dans 64 cm3 d'éther éthylique, ajoute 4 g de 3,3-éthylènedioxy 10β-éthynyl 5α-hydroxy estr 9 (11) èn-17-one, maintient 42 heures au reflux, en ajoutant en 2 fois 96 cm3 du magnésien et 96 cm3 d'éther éthylique. On refroidit le milieu réactionnel, ajoute une solution aqueuse saturée de chlorure d'ammonium, filtre et concentre à sec le filtrat. On chromatographie le résidu sur silice (éluant : cyclohexane-acétate d'éthyle 7-3) et obtient 2,87 g de produit attendu.

Stade B : 10β-éthynyl 17β-hydroxy 17α-/3-(1,1-diméthyléthoxy) propyl/ estra 4,9 (11) dièn-3-one.

On opère comme au stade B de l'exemple 26 à partir de 1,5 g de produit préparé au stade A précédent dans 45 cm3 d'éthanol et 7,5 cm3 d'acide chlorhydrique 5N. On obtient après purification par chromatographie sur silice (éluant : cyclohexane-acétate d'éthyle 7-3) 0,8 g de produit attendu.

Stade C : 10β-éthynyl 17β-hydroxy 17α-(3-hydroxy propyl) estra 4,9 (11) dièn-3-one.

On agite 2 heures 30 minutes à température ambiante sous atmosphère inerte 100 mg de produit obtenu au stade B précédent en solution dans 0,2 cm3 de dioxanne et 0,3 cm3 d'acide chlorhydrique concentré. On verse dans une solution aqueuse saturée de carbonate acide de sodium, extrait à l'acétate d'éthyle, lave la phase organique à l'eau, la sèche et la concentre à sec sous pression réduite. On chromatographie le résidu sur silice (éluant : chlorure de méthylène-méthanol 95-5) et obtient 20 mg de produit attendu. F = 144°C après cristallisation dans l'éther isopropylique puis le 2,2-diméthoxypropane.
$/\alpha/_D$ = +36,5° ± 2° (c = 0,5%, chloroforme).

| Analyse : | | |
|---|---|---|
| Calculé :<br>Trouvé : | C% 77,92<br>77,8 | H% 8,53<br>8,7 |

Exemple 32 : 4',5'-dihydro 10β-éthynyl spiro (estra 4,9 (11) dièn-17,2' (3H) furan)-3-one.

On ajoute en 5 minutes sous atmosphère inerte 122 mg de chlorure de tosyle à une solution de 115 mg de produit préparé comme à l'exemple 31 dans 2,3 cm3 de pyridine et agite 15 heures à température ambiante. On verse dans de l'eau additionnée d'acide chlorhydrique, extrait à l'acétate d'éthyle, lave la phase organique à l'eau, sèche et concentre à sec. On obtient 100 mg de produit brut que l'on purifie par chromatographie sur silice (éluant : cyclohexane-acétate d'éthyle 8-2). F = 126°C après cristallisation dans l'éther isopropylique.
$/\alpha/_D$ = +17° ± 2° (c = 0,5%, chloroforme).

| Analyse : | | |
|---|---|---|
| Calculé :<br>Trouvé : | C% 82,10<br>82,3 | H% 8,38<br>8,6 |

Exemple 33 : 10β-éthynyl 17β-acétoxy 17α-(3-hydroxypropyl) estra 4,9 (11) dièn-3-one.

Stade A : 10β-éthynyl 17β-acétoxy 17α-(3-acétoxypropyl) estra 4,9 (11) dièn-3-on.

On agite 1 heure 30 minutes sous atmosphère inerte 100 mg de produit obtenu comme au stade B précédent, 0,6 cm3 d'anhydride acétique et 5 mg d'acide paratoluènesulfonique. On verse sur de la glace, ajoute une solution aqueuse saturée de bicarbonate de sodium, sèche et concentre à sec sous pression réduite en éliminant l'acide acétique résiduel par entraînement au toluène. On chromatographie le résidu sur silice (éluant : chlorure de méthylène-acétone 9-1) et obtient 98 mg de produit attendu.

Stade B : 10β-éthynyl 17β-acétoxy 17α-(3-hydroxypropyl) estra 4,9 (11) dièn-3-one.

On chauffe au reflux sous atmosphère inerte pendant 1 heure, 70 mg de produit obtenu au stade C précédent, 17 mg de bicarbonate de potassium dans 1 cm3 de méthanol. On verse dans un mélange d'eau et de glace, extrait au chlorure de méthylène, lave la phase organique à l'eau, puis avec une solution aqueuse saturée de chlorure de sodium, sèche et concentre à sec sous pression réduite. On chromatographie le résidu sur silice (éluant : chlorure de méthylène-méthanol 95-5) et obtient 60 mg de produit attendu.
$/\alpha/_D$ = +10° ± 1° (c = 1,2%, chloroforme).

Exemple 34 : 3-oxo 10β-(1-propynyl) 17β-hydroxy 19-nor 17α-pregna 4,9 (11) dièn-21-carboxylate de potassium.

On chauffe au reflux 30 minutes sous atmosphère d'azote 1,69 g de produit préparé à l'exemple 4 dans 21,2 cm3 de potasse méthanolique (0,2 N) et 21,2 cm3 d'eau. On concentre à sec à 50°C sous pression réduite, élimine les traces d'eau par entraînement à l'éthanol. On reprend le résidu à l'éther, essore, lave à l'éther, sèche sous pression réduite à 50°C. On obtient 1,92 g de produit brut. On dissout 1,7 g de ce produit dans 9 cm3 d'éthanol tiède, filtre la solution, ajoute 80 cm3 d'éther, amorce la cristallisation, abandonne à température ambiante puis 1 heure à +4°C, essore et sèche sous pression réduite à 50°C le produit cristallisé. On recueille 1,5 g de produit attendu. F = 248°C.
$/\alpha/_D$ +34,5° ± 2° (c = 0,5% éthanol).

Exemple 35 : 10β-(1-propynyl) 17β-hydroxy 17α-méthyl estra 4,9 (11) dièn-3-one.

Stade A : 3,3-éthylènedioxy 10β-(1-propynyl) 5α,17β-dihydroxy 17α-méthyl estr 9 (11) ène.

Dans 5,6 cm3 d'une solution éthérée d'iodure de méthyl magnésium (0,9M), on ajoute goutte à goutte sous atmosphère inerte 370 mg de 3,3-éthylènedioxy 5α-hydroxy 10β-(1-propynyl) estr-9-èn-17-one préparé comme au stade A de l'exemple 4 en solution dans 4 cm3 de tétrahydrofuranne. On ajoute 10 cm3 de tétrahydrofuranne, agite 1 heure 30 minutes à température ambiante, verse sur du chlorure d'ammonium glacé, extrait à l'acétate d'éthyle, lave la phase organique à l'eau puis avec une solution aqueuse saturée de chlorure de sodium, sèche et concentre à sec sous pression réduite. On chromatographie le résidu sur silice (éluant : cyclohexane-acétate d'éthyle 6-4 à 1°/₀₀ de triéthylamine) et obtient 281 mg de produit attendu. F = 183°C.

Stade B : 10β-(1-propynyl) 17β-hydroxy 17α-méthyl estra 4,9 (11) dièn-3-one.

On chauffe à 50°C pendant 1 heure 30 minutes 596 mg de produit préparé comme au stade A précédent dans 12 cm3 d'éthanol et 3,2 cm3 d'acide chlorhydrique 5N. On ramène à température ambiante, verse dans l'eau glacée, alcalinise avec de l'ammoniaque, extrait à l'acétate d'éthyle, lave la phase organique à l'eau puis avec une solution aqueuse saturée de chlorure de sodium, sèche et concentre à sec sous pression réduite. On chromatographie le résidu sur silice (éluant : n-hexaneacétate d'éthyle 6-4) et obtient 438 mg de produit attendu. F = 130°C après recristallisation dans l'éther.
$/\alpha/_D$ = +95° + 3° (c = 0,3% chloroforme).

| Analyse : | | |
|---|---|---|
| Calculé : | C% 81,44 | H% 8,70 |
| Trouvé : | 81,4 | 8,9 |

Exemple 36 : (17R,2'S) 2'-oxydo 10β-(1-propynyl) spiro /estra 4,9 (11)-dièn-17,5'-1,2-oxathiolane/ 3-one (isomère A) et (17R,2'R) 2'-oxydo 10β-(1-propynyl) spiro /estra 4,9 (11)-dièn-17,5'-1,2-oxathiolane/ 3-one - (isomère B).

Stade A : (17S)-3,3-éthylènedioxy 10β-propynyl spiro /estr 9 (11)-èn-17,2'-oxiran/ 5α-ol.

On ajoute en 10 minutes à température ambiante et sous atmosphère d'azote 16,8 cm3 d'une solution tétrahydrofurannique de terbutylate de potassium (2,4M) à 10 g de 3,3-éthylènedioxy 10β-(1-propynyl) 5α-hydroxy Estr 9 (11) èn-17-one préparé comme au stade A de l'exemple 4 dans 140 cm3 de tétrahydrofuranne, puis ajoute 6,88 g d'iodure de triméthyl sulfonium dans 138 cm3 de diméthyl sulfoxyde et agite pendant 1 heure. On verse sur 200 cm3 d'une solution aqueuse glacée à demi saturée de chlorure d'ammonium, extrait à l'éther, lave la phase organique à l'eau puis avec une solution aqueuse saturée de chlorure de sodium, sèche et concentre à sec. On chromatographie le résidu sur silice (éluant : n-hexane-acétate d'éthyle 7-3 à 1 °/∘∘ de triéthylamine). On obtient 8,79 g de produit attendu.

Stade B : 3,3-éthylènedioxy 10β-éthynyl 5α,17β-dihydroxy 17α-(terbutyl sulfinyl éthyl) estra 9 (11) ène.

On ajoute en 70 minutes à 5 ° C 98,8 cm3 d'une solution (1,6M) de butyllithium dans l'hexane à 19,6 cm3 de méthylbutylsulfoxyde dans 164 cm3 de tétrahydrofuranne et agite 15 minutes. On ajoute en 10 minutes une solution de 3,13 g de produit obtenu au stade A précédent dans 16,2 cm3 de tétrahydrofuranne, agite 10 minutes en maintenant à +5 ° C et laisse revenir à température ambiante. On laisse sous agitation pendant 89 heures, refroidit à 50 ° C ajoute avec précaution 50 cm3 d'une solution aqueuse saturée de chlorure d' ammonium, extrait à l' acétate d'éthyle, lave à l'eau, puis avec une solution aqueuse saturée de chlorure de sodium, sèche et concentre à sec sous pression réduite. On chromatographie le résidu sur silice (éluant : chlorure de méthylène-acétone 6-4 à 1 °/∘∘ de triéthylamine) et isole 1,19 g d'isomère A et 2,3 g d'isomère B (impur).

Stade C :

1) 10β-éthynyl 17β-hydroxy 17α-(terbutyl sulfinyl éthyl) estr 4,9 (11) èn-3-one (isomère A).

On chauffe 1 heure 30 minutes à 50 ° C 1,2 g de l'isomère A obtenu comme au stade B précédent dans 20 cm3 d'éthanol et 20 cm3 d'acide chlorhydrique 5N. On refroidit, ajoute de l'ammoniaque, concentre partiellement, extrait au chlorure de méthylène, lave la phase organique à l'eau puis avec une solution aqueuse saturée de chlorure de sodium, sèche et concentre à sec. On reprend le résidu dans 20 cm3 de méthanol, chauffe au reflux, refroidit, essore et sèche 0,92 g de produit attendu. F = 178 ° C.

2) 10β-éthynyl 17β-hydroxy 17α-(terbutyl sulfinyl éthyl) estr 4,9 (11) èn-3-one (isomère B).

On chauffe 2 heures à 50 ° C 2,3 g de l'isomère B obtenu au stade B précédent dans 68 cm3 d'éthanol et 17 cm3 d'acide chlorhydrique 5N. On refroidit, verse sur de l'eau glacée, extrait à l'acétate d'éthyle, lave la phase organique à l'eau puis avec une solution aqueuse saturée de chlorure de sodium, sèche et concentre à sec. On chromatographie le résidu sur silice (éluant : chlorure de méthylène-acétone 6-4) et obtient 1,266 g de produit attendu que l'on concrète dans l'éther. F = 165 ° -166 ° C.

Stade D :

1) (17R,2'S) 2'-oxydo 10β-(1-propynyl) spiro /estra 4,9 (11)- dièn-17,5'-1,2-oxathiolane/ 3-one (isomère A).

On ajoute 0,415 g de N-chlorosuccinimide dans une solution contenant 1,223 g de l'isomère B obtenu au stade C 2) précédent, 15 cm3 de tétrahydrofuranne et 7 cm3 d'eau. On agite 35 minutes, dilue à l'eau et extrait au chlorure de méthylène. On lave la phase organique à l'eau puis avec une solution aqueuse saturée de chlorure de sodium, sèche et concentre à sec sous pression réduite. On chromatographie le résidu sur sillice (éluant : acétate d'éthyle-cyclohexane 7-3) et obtient 1,06 g de produit brut. On reprend le produit dans 6 cm3 d'éthanol , concentre jusqu'à l'amorce de cristallisation, glace, essore et sèche 0,586 g de produit attendu. F = 210 ° C.
$/\alpha/_D$ = -5,5 ° +2 ° (c = 0,5% chloroforme).

| Analyse : | | | |
|---|---|---|---|
| Calculé :<br>Trouvé : | C% 71,84<br>72,1 | H% 7,34<br>7,4 | S% 8,34<br>8,3 |

2) (17R,2'R) 2'-oxydo 10β-(1-propyntyl spiro /estra 4,9 (11)-dièn-17,5'-1,2-oxathiolane/ 3-one (isomère B).

On opère comme au stade D 1) précédent à partir de 1,068 g de l'isomère A obtenu au stade C 1) dans 15 cm3 de tétrahydrofuranne, 7 cm3 d'eau et de 0,415 g de N-chlorosuccinimide. On obtient 0,480 g de produit attendu. F = 198°C.
$/\alpha/_D$ = +3,5° + 2° (c = 0,51 chloroforme)

| Analyse : | | | |
|---|---|---|---|
| Calculé :<br>Trouvé : | C% 71,84<br>71,5 | H% 7,34<br>7,5 | S% 8,34<br>8,2 |

Exemple 37 : (17S) 3'-propyl 10β-(1-propynyl) spiro /estra 4,9 (11) dièn-17,5'-oxazolidine/ 2',3-dione.

Stade A : 3,3-éthylènedioxy 10β-(1-propynyl) 5α, 17β-dihydroxy 17α-(propylaminométhyl) estr 9 (11) ène.

On chauffe 4 heures à 140°C 3 g de (175) 3,3-éthylènedioxy 10β-(1-propynyl) spiro /estr 9 (11) èn-17,2'-oxiran/ 5α-ol préparé comme au stade A de l'exemple 36 et 68 mg d' acide paratoluène sulfonique dans 4,1 cm3 de toluène et 5,7 cm3 de propylamine. On refroidit dans un bain de glace, extrait au chlorure de méthylène, lave la phase organique avec une solution aqueuse de bicarbonate de sodium, puis à l'eau, sèche et concentre à sec sous pression réduite. On chromatographie le résidu sur silice (éluant : chlorure de méthylène-méthanol-ammoniaque 95-5-0,5) et obtient 3,33 g de produit attendu.

Stade B : 3,3-éthylènedioxy 10β-(1-propynyl) 5α,17β-dihydroxy 17α-/N-propyl N-(éthoxycarbonyl) amino/ méthyl estr 9 (11) ène.

On ajoute en 40 minutes sous atmosphère d'azote 11,5 cm3 d'une solution de chloroformiate d'éthyle dans le chloroforme à 3,3 g de produit obtenu au stade A précédent dans 35 cm3 d'une solution de triéthylamine dans le chloroforme (10-1). On agite 45 minutes, dilue avec une solution aqueuse de bicarbonate de sodium, décante, lave la phase chloroformique à l'eau puis avec une solution aqueuse de chlorure de sodium, sèche et concentre à sec sous pression réduite. On chromatographie le résidu sur silice (éluant : hexane-acétate d'éthyle 6-4 à 1°/∘∘ de triéthylamine) et obtient 3,62 g de produit attendu.

Stade C : 3,3-éthylènedioxy 5α-hydroxy (17S) 3'-propyl 10β'-(1-propynyl) spiro /estr 9 (11) èn 17,5'-oxazolidine 2'-one.

On chauffe au reflux 1 heure sous atmosphère inerte, 3,6 g de produit obtenu au stade B précédent dans 35 cm3 de potasse méthanolique (0,5M). On ajoute de l'eau glacée, ajuste le pH à 6 à l'aide d'acide chlorhydrique N et extrait à l'acétate d'éthyle. On lave la phase organique à l'eau puis àl'aide d'une solution aqueuse saturée de chlorure de sodium, sèche et concentre à sec sous pression réduite. On obtient 3,25 g de produit attendu.

Stade D : (17S) 3'-propyl 10β-(1-propynyl) spiro /estra 4,9 (11) diên-17,5'-oxazolidine/ 2',3-dione.

On opère comme au stade B de l'exemple 35 à partir de 3,2 g de produit obtenu au stade C précédent, 71 cm3 d'éthanol et 18 cm3 d'acide chlorhydrique 5N. On obtient 2,56 g de produit brut que l'on triture dans n-pentane et sèche. On recueille 2,32 g de produit attendu. F = 150°C après recristallisation dans l'éther isopropylique.
$/\alpha/_D$ = -8,5° ± 0,5° (c = 1% chloroforme)

| Analyse : | | | |
|---|---|---|---|
| Calculé :<br>Trouvé : | C% 76,63<br>76,4 | H% 8,16<br>8,3 | N% 3,43<br>3,4 |

Exemple 38 : γ-lactone de l'acide 3-oxo 10β-éthynyl 17β-hydroxy 19-nor pregna 1,4,9 (11) trièn-21-carboxylique.

On chauffe 24 heures au reflux 1 g de produit préparé comme au stade C de l'exemple 1, 50 cm3 de benzène, 1,4 g de dichlorodicyanoquinone et 0,6 g d'acide benzoïque. On refroidit la solution, ajoute 10 cm3 de soude N, extrait à l'acétate d'éthyle. On lave la phase organique à l'eau puis avec une solution aqueuse saturée de chlorure de sodium, sèche et concentre à sec sous pression réduite. On chromatographie le résidu sur silice (éluant : chlorure de méthylène-acétone 95-5) et obtient 508 mg de produit attendu que l'on cristallise dans l'acétate d'éthyle. F = 230 ° C.

Spectre IR (chloroforme)
3305 $cm^{-1}$ : -C≡CH ;
1766 $cm^{-1}$ } -C=O { γ-lactone
1669 $cm^{-1}$ } -C=O { cétone conjuguée ;
1634-1610 $cm^{-1}$ : -C=C- ;
891 $cm^{-1}$ : -C=C- Δ1,4.

Exemple 39 : γ-lactone de l'acide 10β-(2-bromoéthynyl) 17β-hydroxy 3-oxo 19-nor 17α-pregna 4,9 (11) dièn-21-carboxylique.

On ajoute 178 mg de N-bromosuccinimide et 16 mg de nitrate d'argent à une solution de 280 mg de produit obtenu à l'exemple 1 dans 6 cm3 d'acétone. On agite 1 heure à température ambiante, dilue le milieu réactionnel à l'eau, ajoute une solution aqueuse à 10% de thiosulfate de sodium, extrait au chlorure de méthylène, sèche et concentre à sec. On concrète le résidu dans l'éther isopropylique et obtient 250 mg de produit attendu.
$/\alpha/_D$ = +23,5 ° (c = 1% chloroforme).

| Analyse : $C_{23}H_{25}BrO_3$ : 429,36 | | | |
|---|---|---|---|
| Calculé :<br>Trouvé : | C% 64,34<br>64,3 | H% 5,86<br>5,9 | Br% 18,61<br>18,4 |

## Revendications

**1.** Les produits de formule générale (II) :

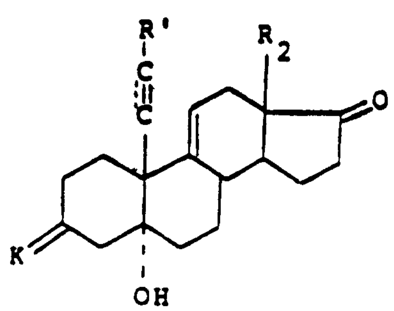

(II)

dans laquelle :

- ou bien R' représente R, R représentant :
- soit un atome d'hydrogène,
- soit un radical alkyle, alkényle ou alkynyle ayant au plus 8 atomes de carbone, éventuellement substitué par les radicaux hydroxyle, carboxy éventuellement estérifié, amino, tritylamino, chloroacétylamino, trifluoroacétylamino ; trichloroéthoxycarbonylamino, monoalkyl ou dialkylamino ou halogènes,
- soit un radical aryle ou aralkyle éventuellement substitué par les radicaux hydroxyle, carboxy éventuellement estérifié, amino, mono ou dialkylamino, alkyle, alkoxy ou alkylthio,
- soit un radical hydroxyle, tétrahydropyrannyloxy, tert-butyloxy, carboxy éventuellement estérifié, amino, mono ou dialkylamino, tritylamino, chloroacétylamino, trifluoroacétylamino ou trichloroéthoxycarbonylamino,
- soit un atome d'halogène,
- soit un radical trialkylsilyle,

ou bien R' représente le substituant R dans lequel les fonctions réactives sont protégées.

$R_2$ représente un radical méthyle ou éthyle.

K représente un groupement protecteur de la fonction cétone, étant entendu que R' ne peut pas représenter un atome d'hydrogène lorque $R_2$ représente un radical méthyle, K représente un radical éthylènedioxy et le trait pointillé sur le substituant R-C≡C- indique la présence d'une troisième liaison entre les carbone qui les portent.

**2.** Les produits de formule générale II telle que définie à la revendication 1 dans laquelle R' est choisi dans le groupe formé par un atone d'hydrogène, un radical alkyle ayant de 1 à 4 atomes de carbone, un radical hydroxyméthyle, un radical tétrahydropyrannyloxyméthyle et un radical phényle et K est choisi dans le groupe formé par les cétals tels que le bis méthoxy, les cétals cycliques tels que l'éthylène dioxy, les oximes et les alkyloximes.

**3.** Procédé de préparation des produits de formule générale (II) telle que définie à la revendication 1 caractérisé en ce :

1- soit l'on fait agir un lithien de formule R'-C≡C-Li sur un produit de formule (III) :

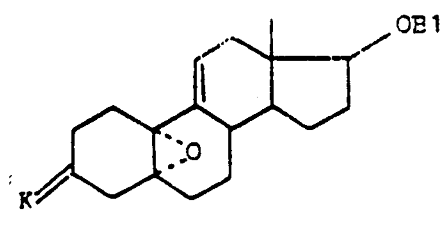

(III)

dans lequel $B_1$ représente un groupement protecteur du radical hydroxyle pour obtenir un produit de formule (IV) :

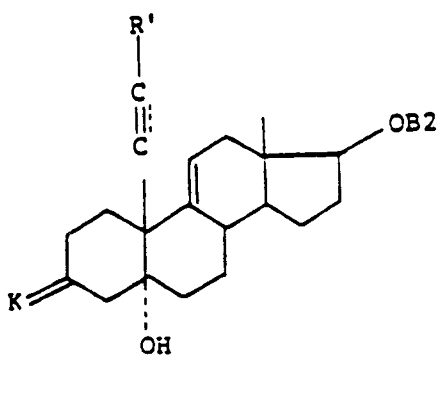

(IV)

dans laquelle $B_2$ représente un atome d'hydrogène ou le radical $B_1$ précédent, produit que l'on soumet d'abord à une réaction de déprotection du radical hydroxyle si le groupement protecteur n'a

pas été clivé lors de la réaction précédente puis à une réaction d'oxydation pour obtenir les produits de formule II ;

2- soit en faisant agir un magnésien de formule R'-C≡C-MgX sur un produit de formule ($III_1$) :

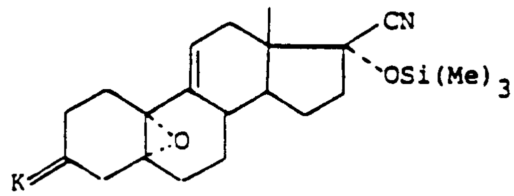

($III_1$)

pour obtenir un produit de formule ($IV_1$) :

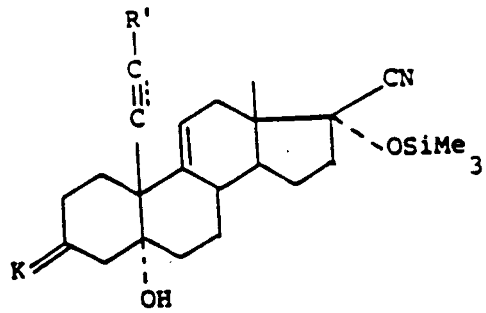

($IV_1$)

produit qui par hydrolyse alcaline donne le produit de formule (II) attendu.

## Claims

**1.** The products of general formula (II):

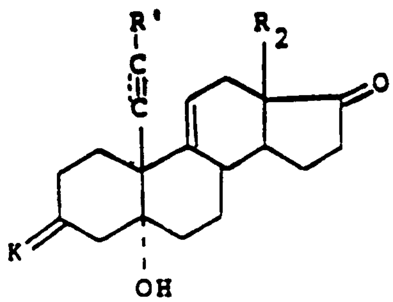

(II)

in which:

- either R' represents R, R representing:
- either a hydrogen atom,
- or an alkyl, alkenyl or alkynyl radical having at most 8 carbon atoms, optionally substituted by the following radicals: hydroxyl, optionally esterified carboxy, amino, tritylamino, chloroacetylamino, trifluoroacetylamino, trichloroethoxycarbonylamino, monoalkyl- or dialkylamino or halogens,
- or an aryl or aralkyl radical optionally substituted by the following radicals: hydroxyl, optionally esterified carboxy, amino, mono- or dialkylamino, alkyl, alkoxy or alkylthio,
- or one of the following radicals: hydroxyl, tetrahydropyrannyloxy, tert-butyloxy, optionally esterified carboxy, amino, mono- or dialkylamino, tritylamino, chloroacetylamino, trifluoroacetylamino or trichloroethoxycarbonylamino,
- or a halogen atom,
- or a trialkylsilyl radical,

or R' represents the substituent R in which the reactive functions are protected.

$R_2$ represents a methyl or ethyl radical.
K represents a protective group of the ketone function, it being understood that R' does not represent a hydrogen atom when $R_2$ represents a methyl radical, K represents an ethylenedioxy radical and the dotted line on the R-C≡C-substituent indicates the presence of a third bond between the carbons which carry them.

2. The products of general formula (II) as defined in claim 1, in which R' is chosen from the group formed by a hydrogen atom, an alkyl radical having 1 to 4 carbon atoms, a hydroxymethyl radical, a tetrahydropyrannyloxymethyl radical and a phenyl radical and K is chosen from the group formed by ketals such as bis methoxy, cyclic ketals such as ethylene dioxy, oximes and alkyloximes.

3. Preparation process for products of general formula (II) as defined in claim 1, characterized in that:
1- either a lithium compound of formula R'-C≡C-Li is reacted on a product of formula (III):

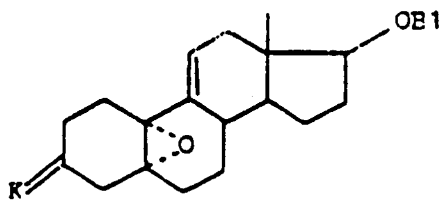

(III)

in which $B_1$ represents a protective group of the hydroxyl radical, in order to obtain a product of formula (IV):

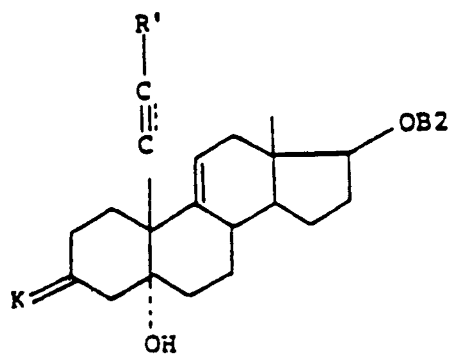

(IV)

in which $B_2$ represents a hydrogen atom or the previous $B_1$ radical, which product is subjected firstly to a deprotection reaction of the hydroxyl radical if the protective group has not been cleaved during the preceding reaction, then to an oxidation reaction in order to obtain the products of formula (II);
2- or by reacting a magnesium compound of formula R'-C = C-MgX on a product of formula ($III_1$):

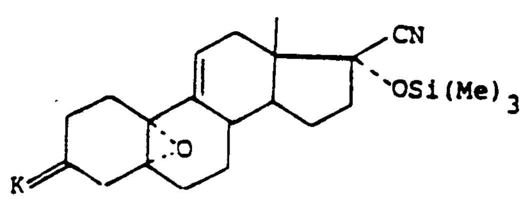

($III_1$)

in order to obtain a product of formula ($IV_1$):

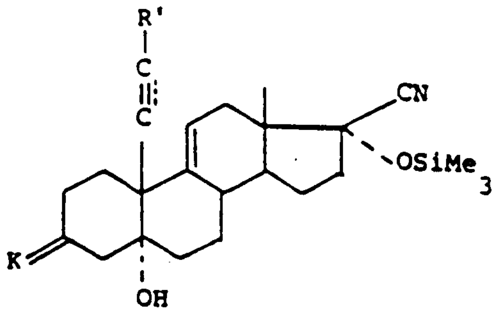

(IV1)

which product gives, by alkaline hydrolysis, the expected product of formula (II).

## Patentansprüche

**1.** Produkte der allgemeinen Formel (II)

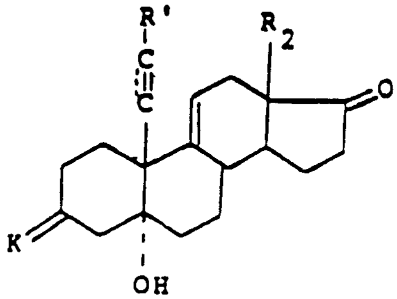

(II)

worin

- entweder R' die Bedeutung von R besitzt, wobei R bedeutet:
  entweder ein Wasserstoffatom,
  oder einen Alkyl-, Alkenyl- oder Alkinylrest mit höchstens 8 Kohlenstoffatomen, gegebenenfalls substituiert durch Hydroxyreste, gegebenenfalls verestertes Carboxy, Amino, Tritylamino, Chloracetylamino, Trifluoracetylamino; Trichlorethoxycarbonylamino, Monoalkyl- oder Dialkylamino oder Halogene,
  oder einen Aryl- oder Aralkylrest, gegebenenfalls substituiert durch Hydroxyreste, gegebenenfalls verestertes Carboxy, Amino, Mono- oder Dialkylamino, Alkyl, Alkoxy oder Alkylthio,
  oder einen Hydroxyl-, Tetrahydropyranyloxy-, tert.-Butyloxy-, gegebenenfalls veresterten Carboxy-, Amino-, Mono- oder Dialkylamino, Tritylamino-, Chloracetylamino-, Trifluoracetylamino- oder Trichlorethoxycarbonylaminorest,
  oder ein Halogenatom,
  oder einen Trialkylsilylrest,
- oder $R^1$ den Substituenten R bedeutet, worin die reaktiven Funktionen geschützt sind,

$R_2$ einen Methyl- oder Ethylrest bedeutet,
K eine Schutzgruppe für die Ketofunktion wiedergibt, mit der Maßgabe, daß R' kein Wasserstoffatom bedeuten kann, wenn $R_2$ für einen Methylrest steht, K eine Ethylendioxygruppe bedeutet und die gestrichelte Linie an dem Substituenten R-C≡.C- die Anwesenheit einer dritten Bindung zwischen den sie tragenden Kohlenstoffatomen anzeigt.

**2.** Produkte der allgemeinen Formel II, wie in Anspruch 1 definiert, worin R' ausgewählt ist unter einem Wasserstoffatom, einem Alkylrest mit 1 bis 4 Kohlenstoffatomen, einer Hydroxymethylgruppe, einer Tetrahydropyranyloxymethylgruppe und einem Phenylrest und K ausgewählt ist unter den Ketalen, wie das Bismethoxyketal, den cyclischen Ketalen, wie das Ethylendioxyketal, den Oximen und den Alkyloximen.

**3.** Verfahren zur Herstellung der Produkte der allgemeinen Formel (II), wie in Anspruch 1 definiert, dadurch gekennzeichnet, daß man

(1) entweder eine Lithiumverbindung der Formel R'-C≡C-Li mit einem Produkt der Formel (III)

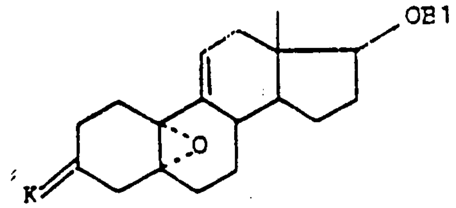

(III)

worin $B_1$ eine Schutzgruppe der Hydroxgruppe wiedergibt, umsetzt, um zu einem Produkt der Formel (IV)

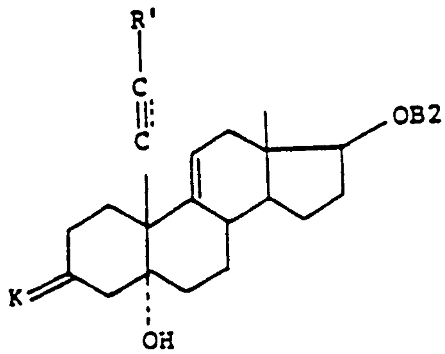

(IV)

worin $B_2$ ein Wasserstoffatom oder den vorstehenden Rest $B_1$ bedeutet, zu gelangen, welches Produkt man zunächst einer Reaktion zur Abspaltung der Schutzgruppe des Hydroxyrestes unterzieht, wenn die Schutzgruppe nicht bei der vorstehenden Reaktion abgespalten worden ist, und anschließend einer Oxidationsreaktion unterzieht, um die Produkte der Formel II zu erhalten;
(2) oder eine Magnesiumverbindung der Formel R'-C≡C-MgX mit einem Produkt der Formel ($III_1$)

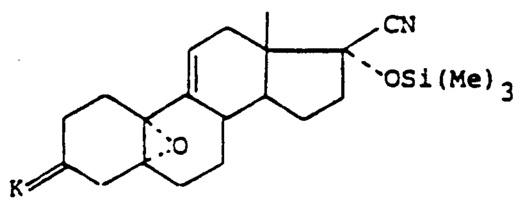

($III_1$)

umsetzt, um ein Produkt der Formel ($IV_1$)

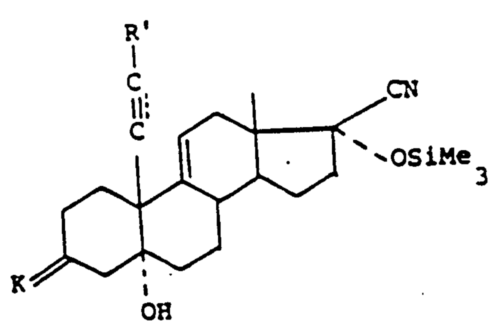

($IV_1$)

zu erhalten, welches Produkt durch alkalische Hydrolyse das erwartete Produkt der Formel (II) ergibt.